(19) Europäisches Patentamt / European Patent Office / Office européen des brevets

(11) EP 3 321 661 A1

(12) **EUROPEAN PATENT APPLICATION**
published in accordance with Art. 153(4) EPC

(43) Date of publication:
16.05.2018 Bulletin 2018/20

(51) Int Cl.:
**G01N 21/27** *(2006.01)*

(21) Application number: 16824241.0

(22) Date of filing: 24.06.2016

(86) International application number:
**PCT/JP2016/068781**

(87) International publication number:
**WO 2017/010261 (19.01.2017 Gazette 2017/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 10.07.2015 JP 2015139139

(71) Applicant: **Sony Corporation**
**Tokyo 108-0075 (JP)**

(72) Inventors:
• **MATSUI, Akira**
**Tokyo 108-0075 (JP)**
• **MURAKAMI, Yoshihiro**
**Atsugi-shi, Kanagawa 243-0812 (JP)**

(74) Representative: **D Young & Co LLP**
**120 Holborn**
**London EC1N 2DY (GB)**

(54) **INSPECTION DEVICE, INSPECTION METHOD, AND PROGRAM**

(57) The present technology relates an inspection apparatus, an inspection method and a program by which accurate measurement light correction can be performed.

The inspection apparatus determines, based on a table in which reference spectral information and a correction gain for correcting measurement spectral information of an inspection object obtained by sensing under a measurement light source are associated with each other and measurement spectral information of a reference reflector plate obtained by sensing under the measurement light source, a measurement light source correspondence correction gain corresponding to the measurement light source, and corrects the measurement spectral information of the inspection object obtained by sensing under the measurement light source based on the determined measurement light source correspondence correction gain. The present technology can be applied, for example, to a vegetation inspection apparatus that measures a vegetation index such as a normalized vegetation index (NDVI).

F I G . 1

EP 3 321 661 A1

**Description**

[Technical Field]

The present technology relates an inspection apparatus, an inspection method and a program, and particularly to an inspection apparatus, an inspection method and a program by which accurate measurement light correction can be performed.

[Background Art]

Conventionally, it is known to use, when measurement of an inspection index of an inspection object such as a plant is performed, a reference reflector plate in order to correct a variation of a measurement light source upon measurement (refer to, for example, PTL 1) .

[Citation List]

[Patent Literature]

[PTL 1]
JP 2006-101768A

[Summary]

[Technical Problem]

However, with the conventional technology, upon measurement of an inspection index of an inspection object, a variation of a measurement light source sometimes makes it difficult to perform accurate measurement light correction. Therefore, it is demanded to perform, upon measurement of an inspection index of an inspection object, accurate measurement light correction even if the measurement light source varies.

The present technology has been made in view of such a situation as described above and makes it possible to perform accurate measurement light correction by removing the light source dependency upon measurement of an inspection index of an inspection object.

[Solution to Problem]

The inspection apparatus according to one aspect of the present technology is an inspection apparatus including a correction gain determination unit configured to determine, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source, and a correction unit configured to correct the measurement spectral information of the inspection object obtained by sensing under the measurement light source based on the determined measurement light source correspondence correction gain.

The inspection apparatus of the one aspect of the technology may be an independent apparatus or an internal block that configures one apparatus. Further, an inspection method or a program of the one aspect of the present technology is an inspection method or a program that corresponds to the inspection apparatus of the one aspect of the present technology described above.

In the inspection apparatus, inspection method and program of the one aspect of the present technology, based on the table in which reference spectral information and a correction gain for correcting measurement spectral information of an inspection object obtained by sensing under a measurement light source are associated with each other and measurement spectral information of the reference reflector plate or the reference transmission plate obtained by sensing under the measurement light source, the measurement light source correspondence correction gain corresponding to the measurement light source is determined, and the measurement spectral information of the inspection object obtained by sensing under the measurement light source is corrected based on the determined measurement light source correspondence correction gain.

[Advantageous Effect of Invention]

With the one aspect of the present technology, accurate measurement light correction can be performed.
It is to be noted that the effect described here is not necessarily restrictive but may be any effect described in the present disclosure.

[Brief Description of Drawings]

[FIG. 1]

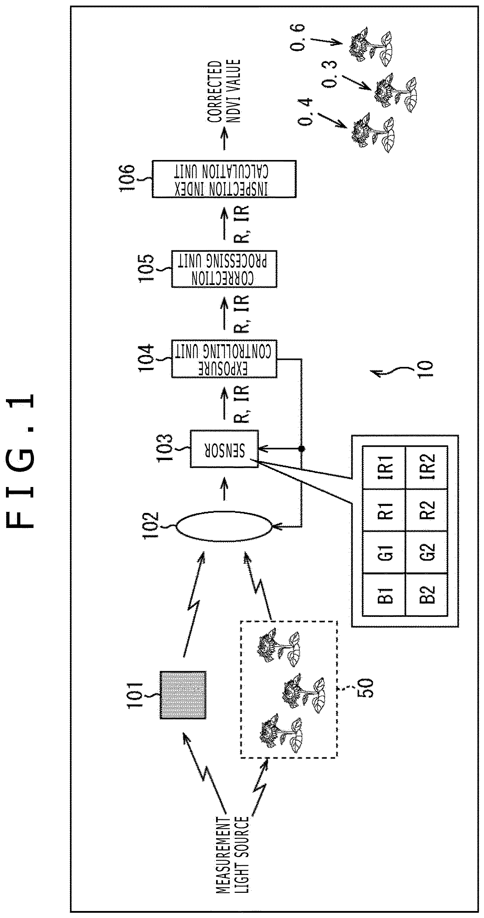

FIG. 1 is a view depicting an example of a configuration of a vegetation inspection apparatus to which the present technology is applied.
[FIG. 2]

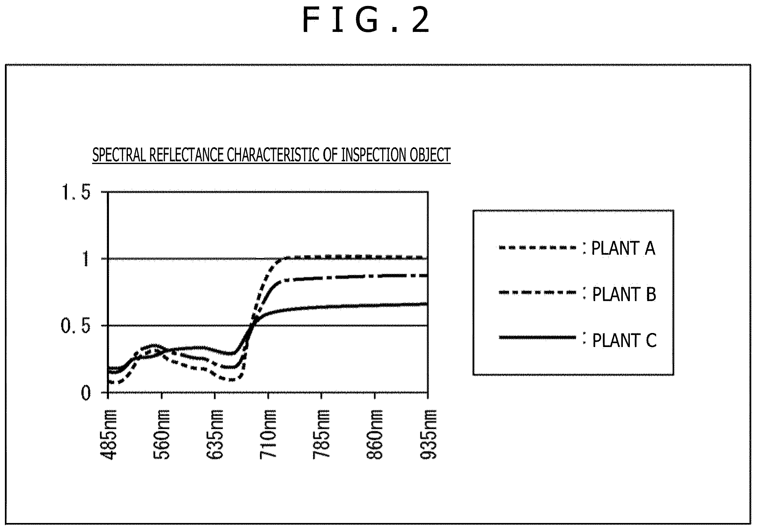

FIG. 2 is a view depicting a spectral reflectance characteristic of an inspection object.
[FIG. 3]

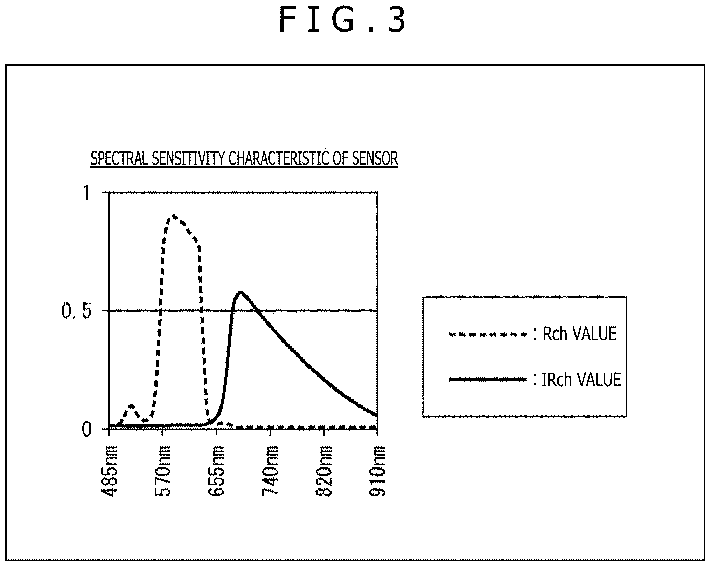

FIG. 3 is a view depicting a spectral sensitivity characteristic of a sensor.
[FIG. 4]

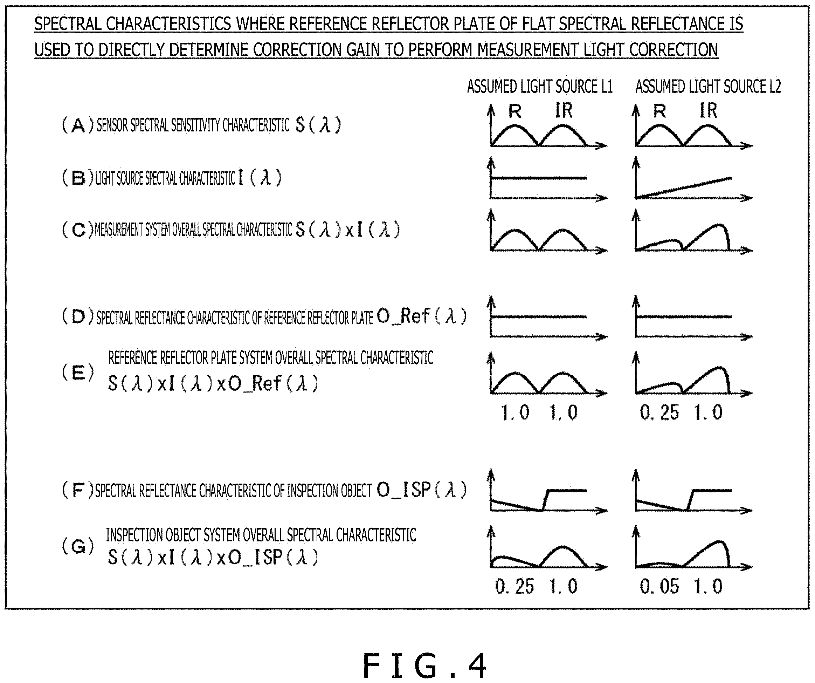

FIG. 4 is a view depicting spectral characteristics where a reference reflector plate of a flat spectral reflectance is used to directly determine a correction gain and perform measurement light correction.
[FIG. 5]

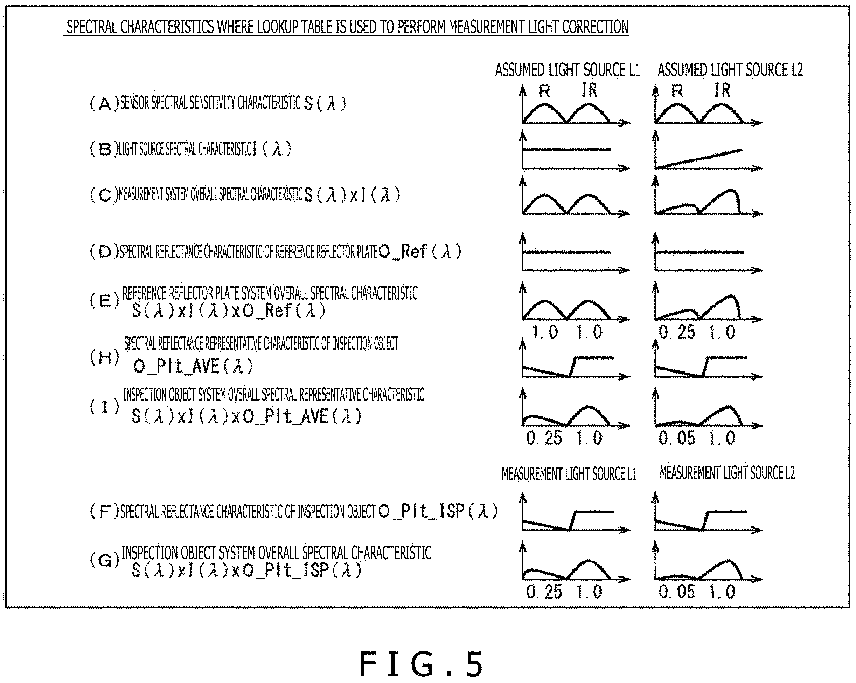

FIG. 5 is a view depicting spectral characteristics where a lookup table (LUT) is used to perform measurement light correction.
[FIG. 6]

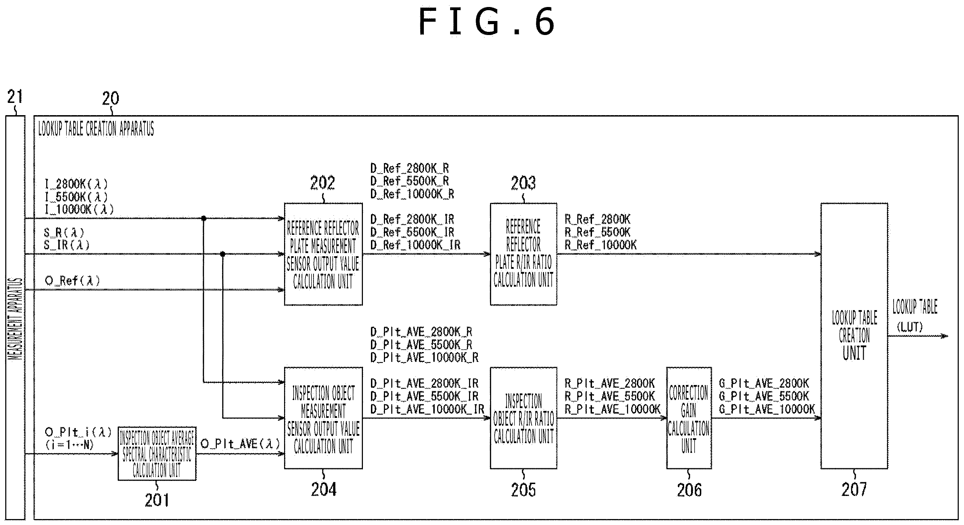

FIG. 6 is a view depicting a functional configuration of a lookup table creation apparatus.
[FIG. 7]

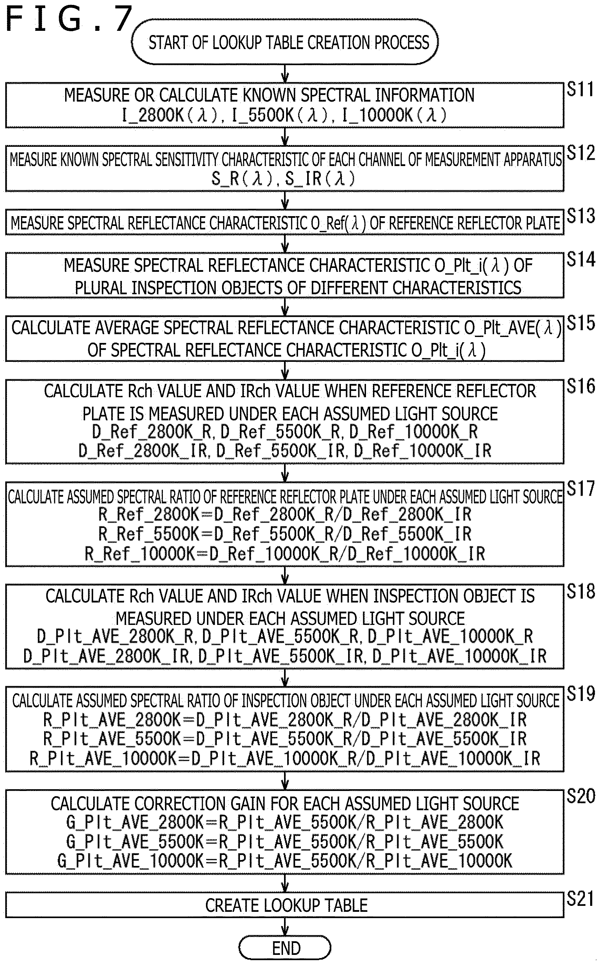

FIG. 7 is a flow chart illustrating the lookup table creation process.
[FIG. 8]

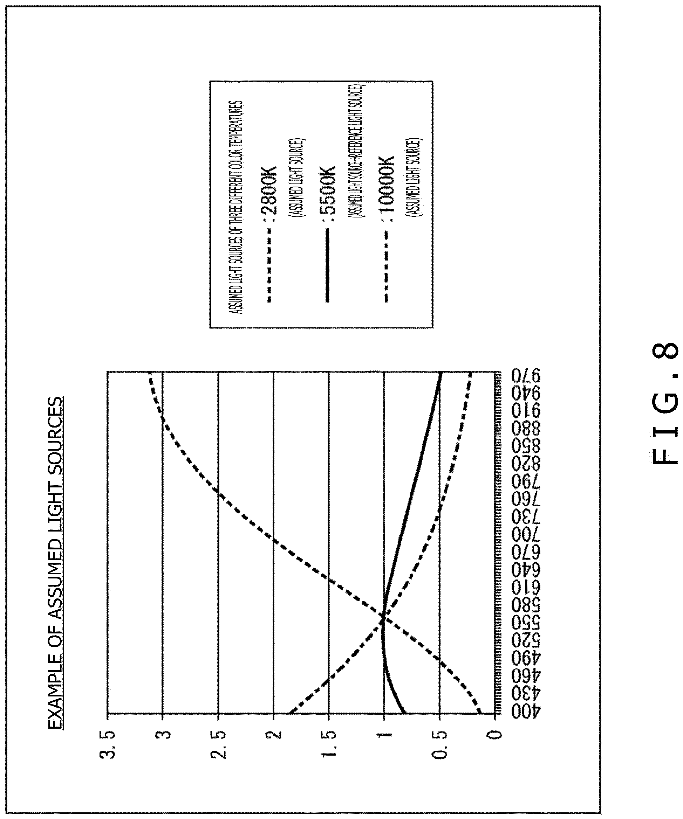

FIG. 8 is a view depicting an example of an assumed light source.
[FIG. 9]

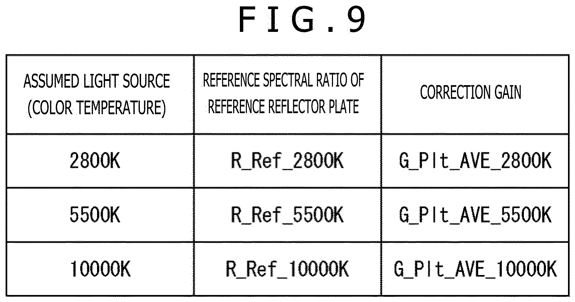

FIG. 9 is a view depicting an example of a lookup table (LUT).
[FIG. 10]

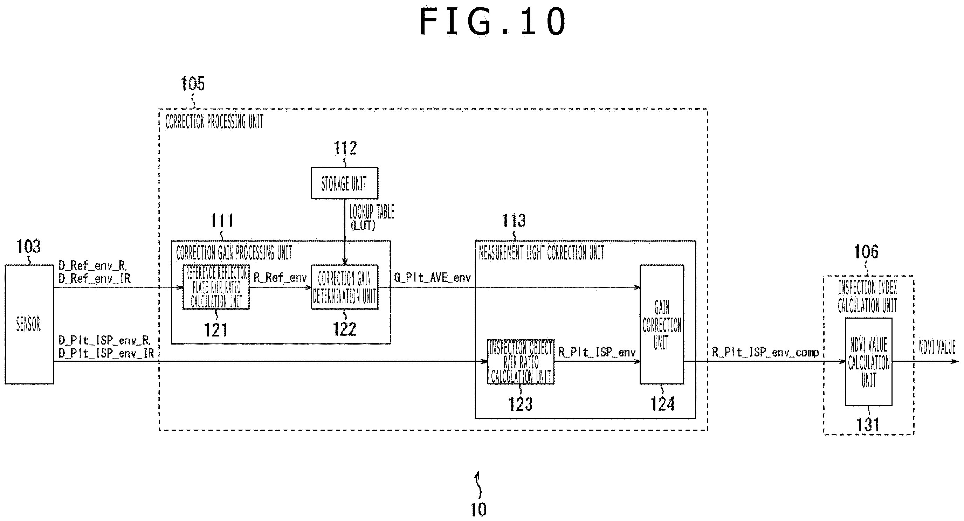

FIG. 10 is a view depicting a first functional configuration of the vegetation inspection apparatus upon measurement of an inspection index.
[FIG. 11]

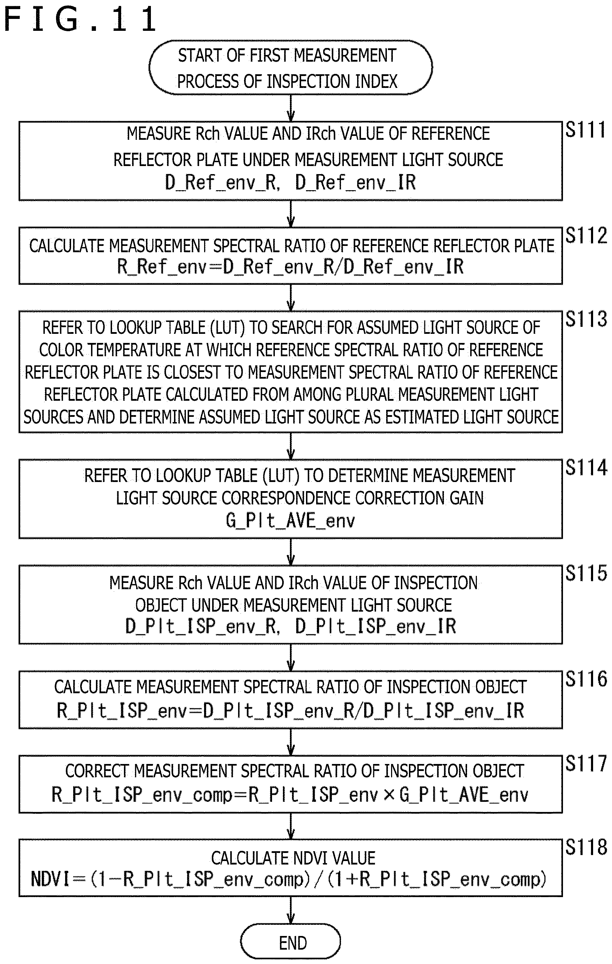

FIG. 11 is a flow chart illustrating a first measurement process of an inspection index.
[FIG. 12]

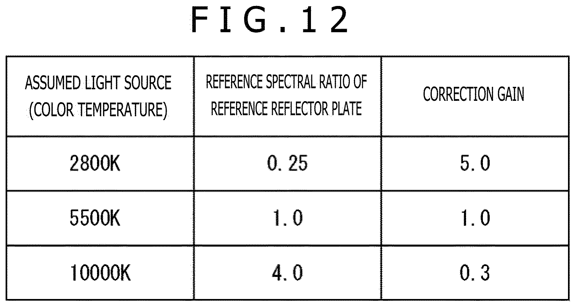

FIG. 12 is a view depicting an example of particular numerical values of the lookup table (LUT).
[FIG. 13]

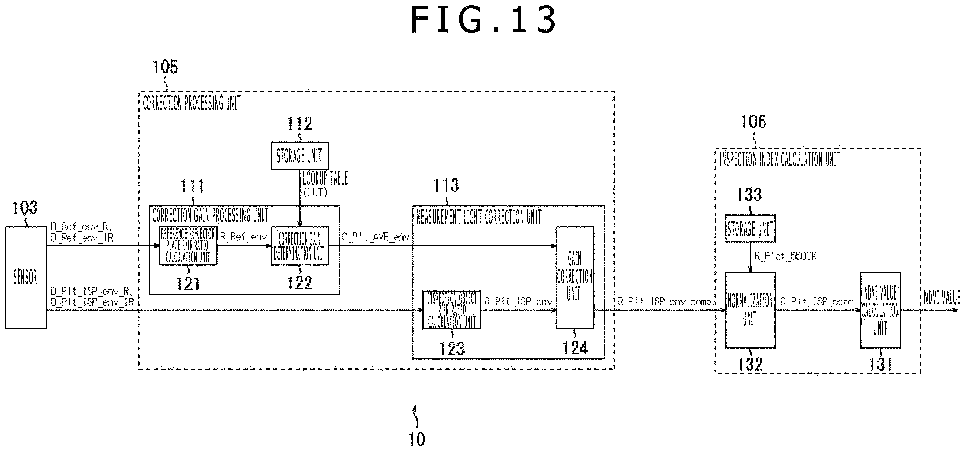

FIG. 13 is a view depicting a second functional configuration of the vegetation inspection apparatus upon measurement of an inspection index.
[FIG. 14]

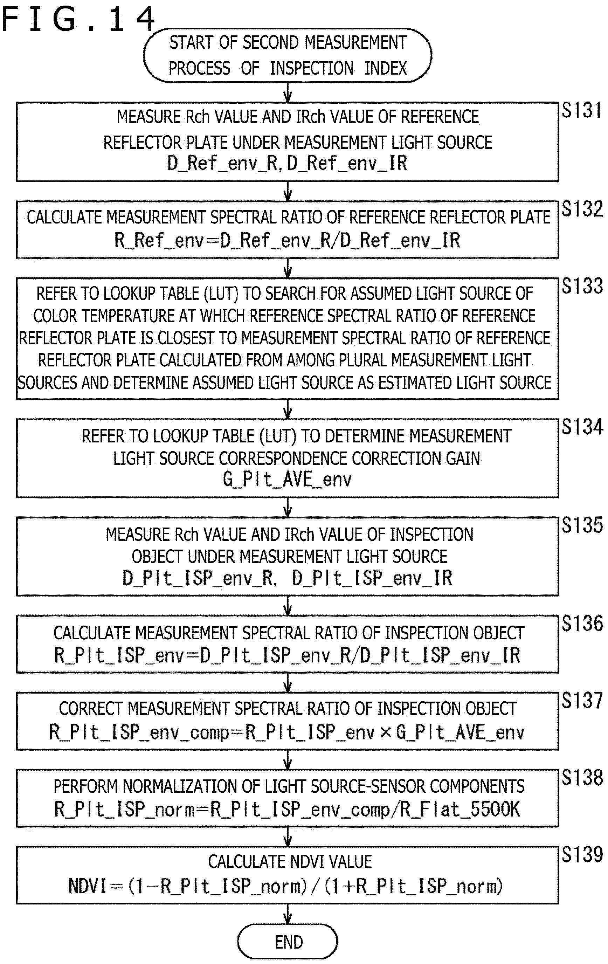

FIG. 14 is a flow chart illustrating a second measurement process of an inspection index.
[FIG. 15]

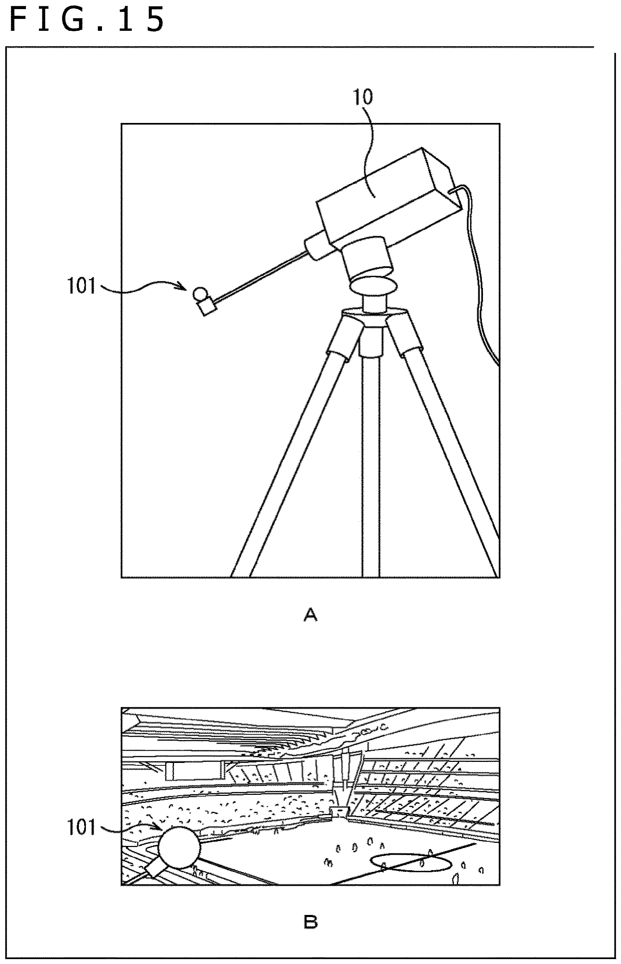

FIG. 15 is a view depicting an example of a case in which a reference reflector plate is attached in front of a camera.
[FIG. 16]

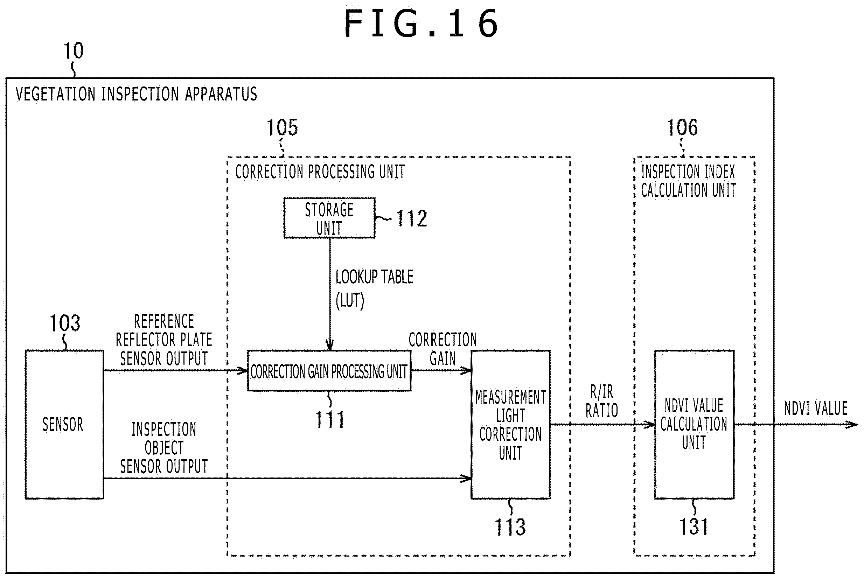

FIG. 16 is a view depicting an example of a configuration of the vegetation inspection apparatus.
[FIG. 17]

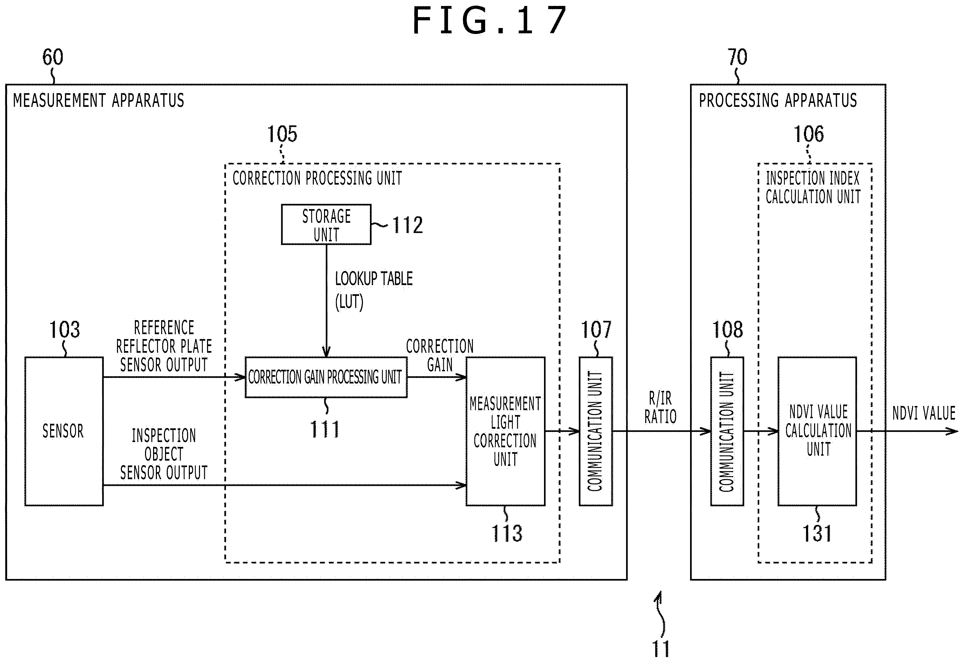

FIG. 17 is a view depicting a first example of a configuration of a vegetation inspection system.
[FIG. 18]

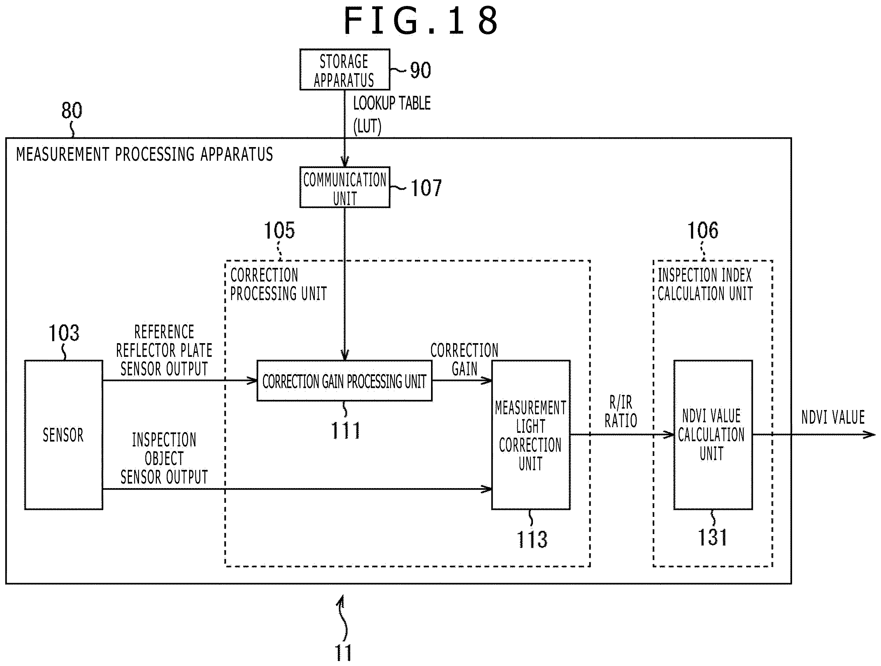

FIG. 18 is a view depicting a second example of a configuration of the vegetation inspection system.
[FIG. 19]

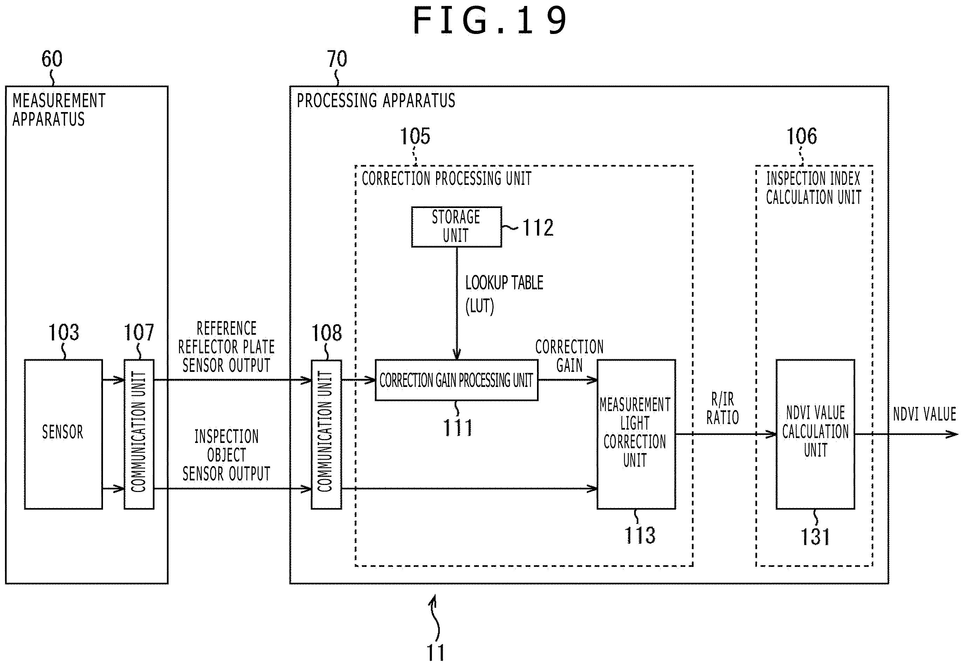

FIG. 19 is a view depicting a third example of a configuration of the vegetation inspection system.
[FIG. 20]

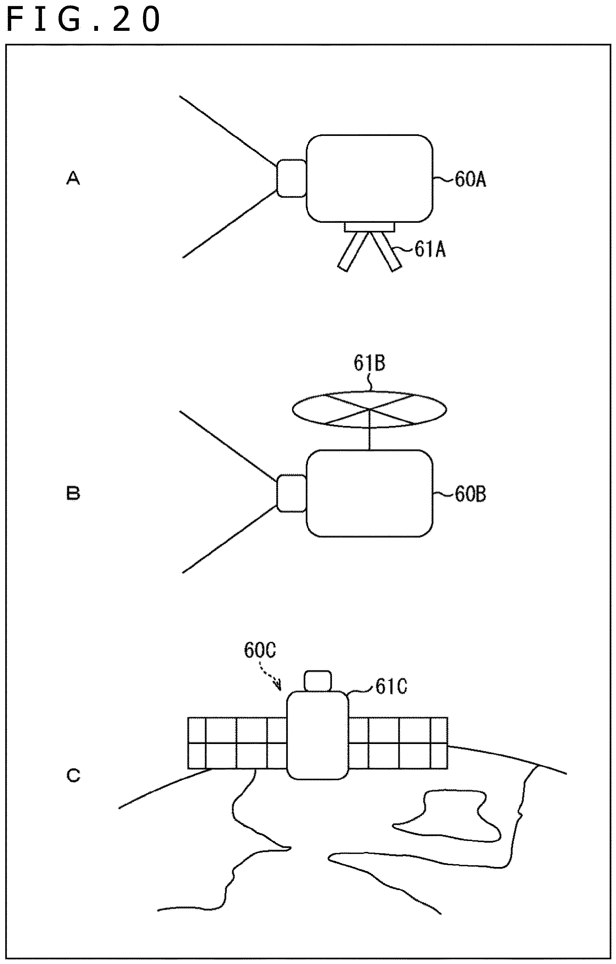

FIG. 20 is a view depicting a particular example of a measurement apparatus.
[FIG. 21]

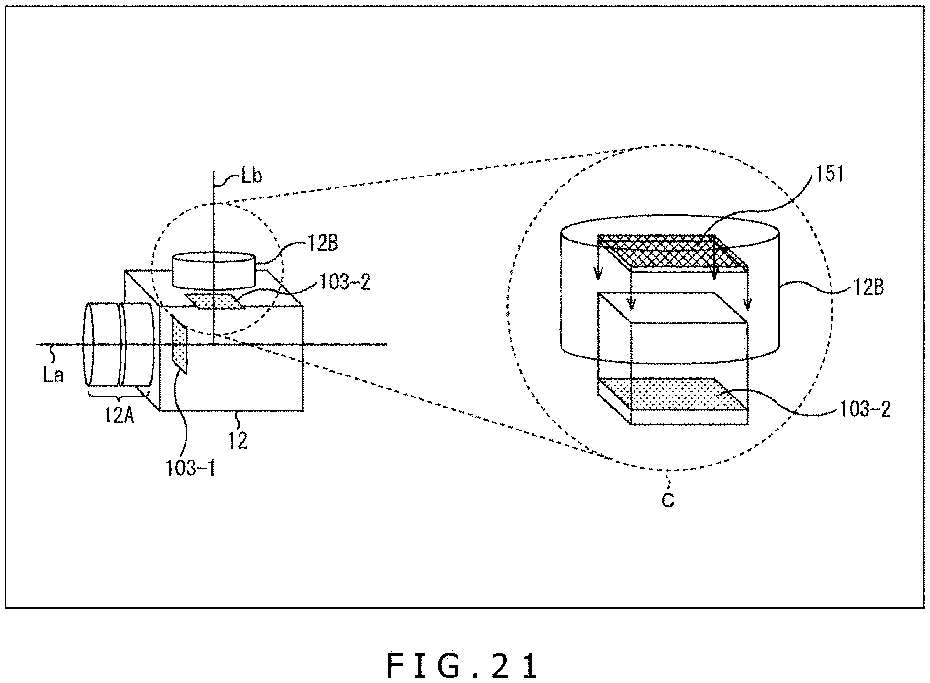

FIG. 21 is a perspective view depicting an example of a configuration of an appearance of a vegetation inspection apparatus where a monocular configuration is adopted.

[FIG. 22]

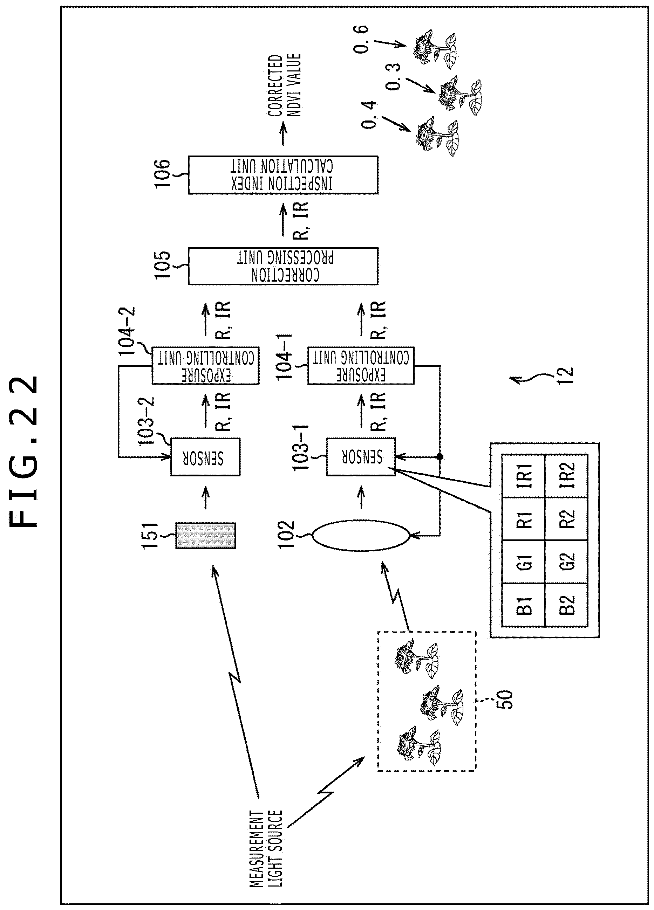

FIG. 22 is a view depicting an example of a configuration of the vegetation inspection apparatus where a monocular configuration is adopted.
[FIG. 23]

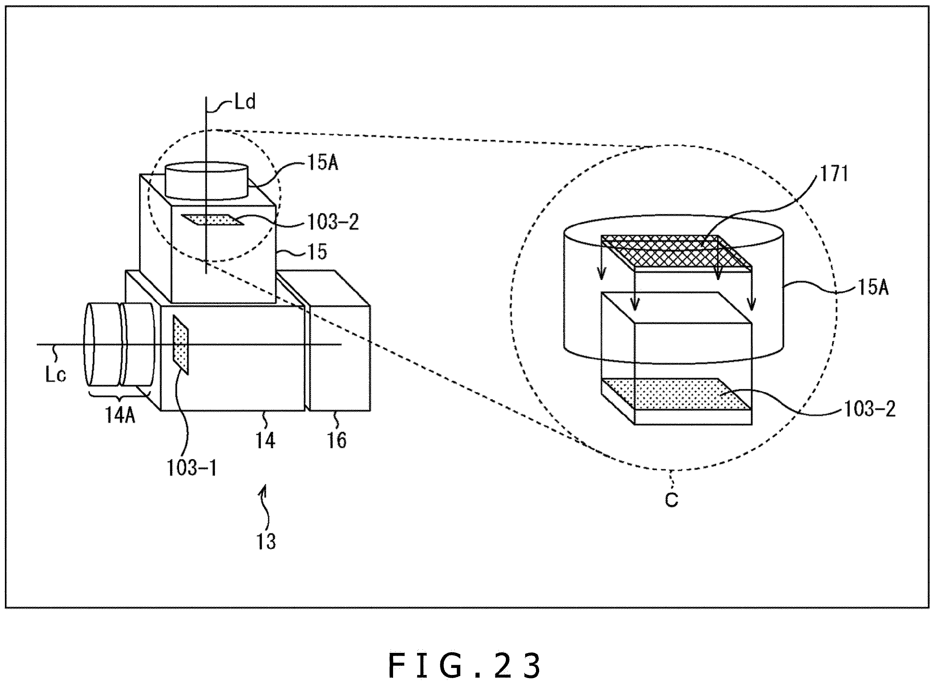

FIG. 23 is a perspective view depicting an example of a configuration of an appearance of a vegetation inspection apparatus where a compound eye configuration is adopted.
[FIG. 24]

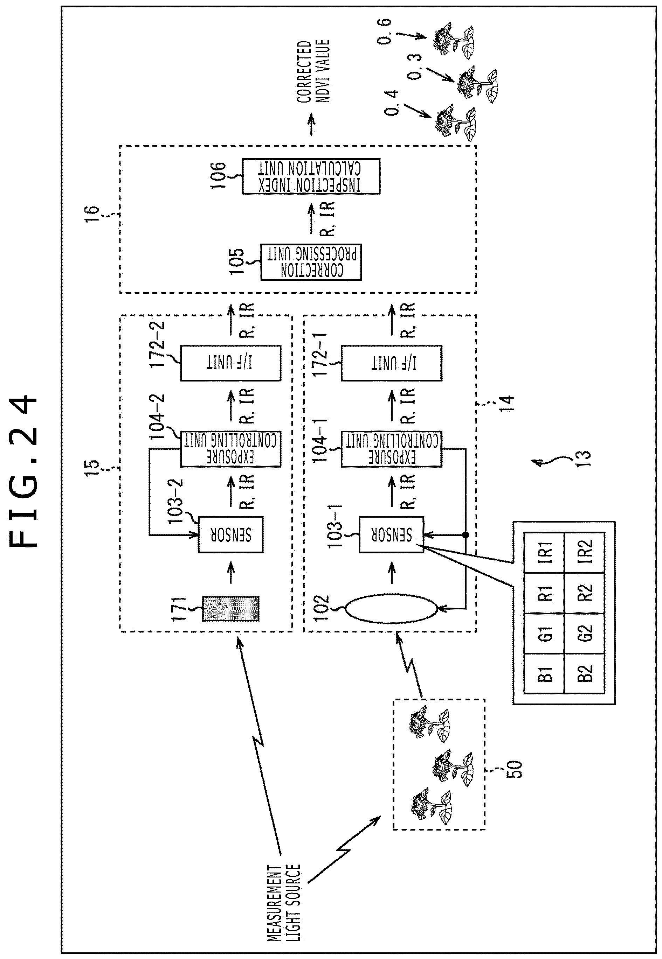

FIG. 24 is a view depicting an example of a configuration of a vegetation inspection apparatus where a compound eye configuration is adopted.
[FIG. 25]

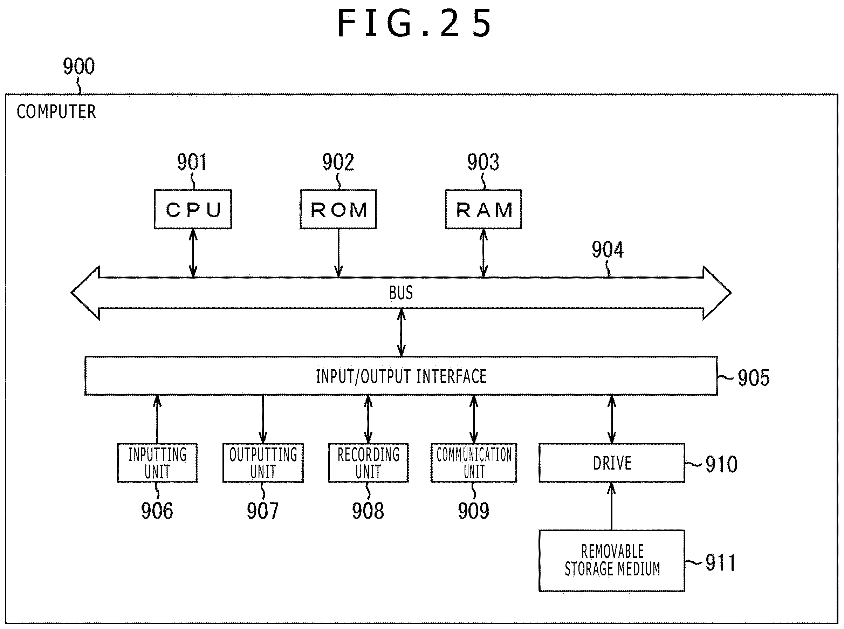

FIG. 25 is a view depicting an example of a configuration of a computer.

[Description of Embodiment]

In the following, an embodiment of the present technology is described with reference to the drawings. It is to be noted that the description is given in the following order.
1. Configuration of System
2. Principe of Inspection Where Lookup Table (LUT) Is Used
3. Creation of Lookup Table (LUT)
4. Inspection of Inspection Object
5. Modifications
6. Configuration of Computer

<1. Configuration of System>

(Configuration of vegetation inspection apparatus)

FIG. 1 is a view depicting an example of a configuration of a vegetation inspection apparatus 10 to which the present technology is applied. It is to be noted that, in the description of FIG. 1, graphs of FIGS. 2 and 3 are suitably referred to.
The vegetation inspection apparatus 10 of FIG. 1 is an apparatus that performs sensing of an inspection object 50 such as a plant to perform measurement of an inspection index of the inspection object 50. Here, the term sensing signifies to measure an object. Further, the sensing includes to pick up an image of an object (image pickup object), and also a case in which sensing of light of a wavelength other than those of visible rays is performed is included in image pickup.
Referring to FIG. 1, the vegetation inspection apparatus 10 is configured from a reference reflector plate 101, a lens 102, a sensor 103, an exposure controlling unit 104, a correction processing unit 105, and an inspection index calculation unit 106. It is to be noted that the reference reflector plate 101 is installed at a predetermined position at which it can be measured (sensed) solely or simultaneously with the inspection object 50 by the vegetation inspection apparatus 10. It is to be noted that, in FIG. 1, the exposure controlling unit 104, correction processing unit 105, inspection index calculation unit 106 and so forth may be configured including circuitry.
In FIG. 1, a case is described in which, as the inspection object 50, a plant is an object, and a vegetation index is calculated as an inspection index of the plant. The vegetation index is an index indicative of a distribution situation or an activity of vegetations. In the following description, as the vegetation index, a normalized vegetation index (NDVI: Normalized Difference Vegetation Index) is described.
Further, in the following description, the term spectral ratio is a value representative of a relationship of spectra obtained by dispersing light from an object. Here, in order to determine a normalized vegetation index (NDVI value), since a value of an R (red) component and a value of an IR (infrared) component are required as a spectroscopic spectrum, the spectral ratio is a value representative of a relationship between an R component and an IR component.
As the spectral ratio in this instance, data may be of any type if the data represents a ratio of R and IR or values from which a ratio of R and IR can be determined such as, for example, a value obtained by calculating R/IR, a value obtained by calculating IR/R, or values of R and IR retained independently of each other. In the following description, a case in which, as the spectral ratio, a value obtained by calculating R/IR (hereinafter referred to also as "R/IR ratio") is used is described as an example.
Further, while a lookup table (LUT: Lookup Table) created in advance is used in the vegetation inspection apparatus 10, a light source assumed in advance upon creation of a lookup table (LUT) is referred to as assumed light source, and a light source of a reference color temperature from among a plurality of assumed light sources having different color temperatures from each other is referred to as reference light source. Furthermore, a light source that illuminates an object upon measurement of an inspection index of an inspection object 50 under various light sources is referred to as measurement light source.

Under the measurement light source, reflection light from the inspection object 50 enters the sensor 103 through the lens 102.

FIG. 2 is a view depicting spectral reflectance characteristics of plants as the inspection object 50. In FIG. 2, the axis abscissa represents the wavelength (nm) and the axis of ordinate represents the reflectance. Further, a plant A, another plant B and a further plant C are plants of the same type (for example, turf) but are different in vegetation representative of the amount or the activity of the plants. As depicted in FIG. 2, the spectral reflectance characteristic differs among the plants of different vegetations.

Further, under the measurement light source, reflection light from the reference reflector plate 101 enters the sensor 103 through the lens 102.

The sensor 103 is configured, for example, from a spectroscope having a plurality of optical filters that transmit light of predetermined wavelength bands, and a sensing element including a plurality of pixels arrayed in a matrix on a sensor face thereof and has a spectroscopic sensing function. The sensor 103 disperses, by the spectroscope thereof, light (reflection light) from objects (inspection object 50 and reference reflector plate 101) incident thereto through the lens 102 and detects, by the sensing element thereof, light irradiated upon the sensor face to output measurement signals (measurement values) according to the brightness values of the respective spectral components.

As depicted in FIG. 1, in the spectroscope of the sensor 103, for example, 8 pixels with length × width of 2 × 4 are set as one set, and eight different optical filters that pass light of wavelength bands different from each other are arranged corresponding to the pixels configuring the one set. In particular, corresponding to 8 pixels of one set, an optical filter that passes first blue light B1, an optical filter that passes second blue light B2, an optical filter that passes first blue light G1, an optical filter that passes second blue light G2, an optical filter that passes first red light R1, an optical filter that passes second red light R2, an optical filter that passes first infrared light IR1 and an optical filter that passes second infrared light IR2 are arranged in an ascending order of the wavelength.

Further, the spectroscope is configured, defining such optical filters of 8 pixels as described above as one set, from n sets (n is a natural number equal to or greater than 1) optical filters arranged successively over an overall area of the sensor face of the sensing element. It is to be noted that the set of optical filters is not limited to the configuration in which 8 pixels are set as one set, but different forms such as, for example, a configuration in which four pixels are set as one set can be adopted.

Here, where a normalized vegetation index (NDVI value) is measured as the vegetation index, since measurement values of the R (red) component and the IR (infrared) component are required, the sensor 103 measures (senses) a value of the R component (R channel (Rch) value) and a value of the IR component (IR channel (IRch) value) in response to light (reflection light) from objects and supplies the values to the exposure controlling unit 104 and the correction processing unit 105.

It is to be noted that the value of the R component (R channel value) is a measurement value corresponding, for example, to the optical filter that passes the first red light R1 or a measurement value corresponding to the optical filter that passes the second red light R2. Meanwhile, the value of the IR component (IR channel value) is a measurement value corresponding, for example, to the optical filter that passes the first infrared light IR1 or a measurement value corresponding to the optical filter that passes the second infrared light IR2.

FIG. 3 is a view depicting a spectral sensitivity characteristic of the sensor 103. Referring to FIG. 3, the axis of abscissa represents the wavelength (nm) and the axis of ordinate represents the spectral sensitivity. Here, it is known that, if the inspection object 50 is a plant, then the reflection in the visible region is small and the reflection in the near infrared region is great. This is because the spectral reflectance characteristic has a relationship with photosynthesis of a plant such that wavelengths in the visible region that is effective for photosynthesis are absorbed to decrease the reflection from the visible region while the reflection is relatively great in the near infrared region. The sensor 103 has the spectral sensitivity characteristic depicted in FIG. 3 in order to cope with such a spectral reflection characteristic of a plant.

It is to be noted that, for the sensor 103, not only an area sensor that captures an object by plane but also a line sensor that captures an object by line can be used. Further, even where only one pixel is arranged as each of a pixel for the R component and a pixel for the IR component on the sensing element, an object can be scanned if a mechanism for moving the sensor or the measurement object is provided.

The exposure controlling unit 104 controls the shutter speed of an optical system and the aperture amount by an iris (aperture) of the lens 102 and so forth, the shutter speed of an electronic shutter of the sensor 103 or the like such that measurement (sensing) is performed in a state in which signal charge remains within a dynamic range without being saturated in the sensor 103 to perform exposure control.

The correction processing unit 105 performs, on the basis of the R channel value and the IR channel value supplied thereto from the sensor 103, a correction process for correcting the spectral ratio (R/IR ratio) between the R channel value and the IR channel value and supplies information indicative of the spectral ratio (R/IR ratio) after correction to the inspection index calculation unit 106.

In this correction process, a lookup table (LUT) created in advance is referred to to determine a correction gain of a spectral ratio according to a measurement spectral ratio (R/IR ratio) of the reference reflector plate 101 obtained by measurement (sensing) under a measurement light source (such correction gain is hereinafter referred to as measurement light source correspondence correction gain), and the measurement light source correspondence correction gain is used to correct the measurement spectral ratio (R/IR ratio) of the inspection object 50 obtained by measurement (sensing) under the measurement light source.

It is to be noted that, in the lookup table (LUT), a reference spectral ratio (R/IR ratio) and a correction gain are associated with each other for each assumed light source of each color temperature. It is to be noted that the reference spectral ratio corresponds to the assumed spectral ratio (R/IR ratio) of the reference reflector plate 101 under the assumed light source. Further, the correction gain is a gain for correcting the measurement spectral ratio (R/IR ratio) of the inspection object 50 obtained by measurement (sensing) under the measurement light source. It is to be noted that the particular substance of the creation method of a lookup table (LUT) is hereinafter described with reference to FIGS. 6 to 9.

The inspection index calculation unit 106 calculates and outputs a vegetation index using the information supplied from the correction processing unit 105 and indicative of the measurement spectral ratio (R/IR ratio) of the inspection object 50 after correction. Here, the (corrected) NDVI value can be determined as a vegetation index by arithmetically operating an expression (1) given below.

$$NDVI = (IR - R)/(IR + R) = (1 - R/IR)/(1 + R/IR) \ ... \ (1)$$

It is to be noted that, in the expression (1), IR represents the reflectance in the near infrared region and R represents the reflectance in the visible region (red). It is to be noted that the detailed substance of the inspection method of the inspection object 50 such as to calculate a normalized vegetation index (NDVI value) is hereinafter described with reference to FIGS. 10 to 14.

The vegetation inspection apparatus 10 is configured in such a manner as described above. The vegetation inspection apparatus 10 of FIG. 1 uses a lookup table (LUT) created in advance to determine a measurement light source correspondence correction gain according to a measurement spectral ratio (R/IR ratio) of the reference reflector plate 101 under the measurement light source, corrects the measurement spectral ratio (R/IR ratio) of the inspection object 50 under the measurement light source in response to the determined measurement light source correspondence correction gain, and calculates an inspection index (NDVI value) from the measurement spectral ratio (R/IR ratio) after corrected.

Consequently, for example, if 0.6 is measured as the NDVI value of the inspection object 50 (specific plant) under the measurement light source by the vegetation inspection apparatus 10 of FIG. 1, then the NDVI value of 0.6 corresponds to the NDVI value of the inspection object 50 under the reference light source, and even if the light source varies by a variation of the weather such as a fine, cloudy or rainy weather, since the light source dependency is removed, 0.6 is measured as the NDVI value if the object is the same inspection object 50 (specific plant). Further, this applies also to a case in which a value of, for example, 0.3 or 0.4 is measured as the NDVI value of a different inspection object 50 of a different vegetation under the measurement light source, and such values correspond to the NDVI values of the inspection object 50 under the reference light source and are measured as values from which the light source dependency is removed. On the other hand, different from the vegetation inspection apparatus 10, if the light source dependency is not removed, then if the light source varies by a change of the weather such as, for example, a fine weather or a cloudy weather, then even if the object is the same inspection object (plant), the NDVI value is measured as a different value.

<2. Principe of Inspection Where Lookup Table (LUT) Is Used>

Here, in what manner spectral characteristics of a light source and an object (inspection object 50 and reference reflector plate 101) act on the output value of the sensor 103, the spectral ratio (R/IR ratio) and the measurement light correction is indicated with reference to FIGS. 4 and 5 to describe a principle of an inspection performed by the vegetation inspection apparatus 10.

It is to be noted here that a case is described first in which, using a reference reflector plate of a white plate or a gray plate that has a flat spectral reflectance characteristic and is normally used for white balance in an RGB camera or the like (such reference reflector plate is hereinafter referred to also as flat reference reflector plate 101F), a correction gain is determined directly from an output value of the sensor 103 when the flat reference reflector plate 101F is measured to perform measurement light correction with reference to FIG. 4 for comparison. Thereafter, a case in which measurement light correction is performed using a lookup table (LUT) is described with reference to FIG. 5.

(Case in which flat reference reflector plate is used to directly determine correction gain to perform measurement light correction)

FIG. 4 is a view illustrating a case in which the flat reference reflector plate 101F is used as the reference reflector plate to directly determine a correction gain to perform measurement light correction. It is to be noted that, in FIG. 4, the axis of abscissa of graphs represents the wavelength and the axis of ordinate represents the intensity.

In the graphs of an assumed light source L1 and an assumed light source L2 of the sensor spectral sensitivity characteristic $S(\lambda)$ of A of FIG. 4, the left side waveform represents a spectral sensitivity characteristic of the R channel and the right side waveform represents a spectral sensitivity characteristic of the IR channel. It is to be noted that the intensity of the axis of ordinate of A of FIG. 4 represents the intensity of light measured by the sensor 103.

In the light source spectral characteristic $I(\lambda)$ of B of FIG. 4, the graph of the assumed light source L1 represents a characteristic in the case in which the spectral characteristic is flat, and the graph of the assumed light source L2 represents a characteristic that, as the wavelength increases, the spectral characteristic increases in intensity. It is to be noted that the intensity of the axis of ordinate of B of FIG. 4 represents an intensity of light radiated by each light source.

The measurement system overall spectral characteristic of C of FIG. 4 represents $S(\lambda) \times I(\lambda)$, namely, a spectral characteristic that is the product of the sensor spectral sensitivity characteristic $S(\lambda)$ of A of FIG. 4 and the light source spectral characteristic $I(\lambda)$ of B of FIG. 4. This represents an overall spectral characteristic of the measurement system taking both a characteristic of the light source and a characteristic of the sensor under the different light sources into consideration. It is to be noted that the intensity of the axis of ordinate of C of FIG. 4 represents an intensity obtained by multiplying the light intensity of A of FIG. 4 and the light intensity of B of FIG. 4.

It is assumed that, in the following description, measurement of an object such as the reference reflector plate 101F or the inspection object 50 is assumed and the description is given using an R channel value and an IR channel value that are output values of the sensor 103. Here, the output values of the sensor 103 vary in proportion to a value obtained by multiplying the measurement system overall spectral characteristic $S(\lambda) \times I(\lambda)$ and $O\_Ref(\lambda)$ or $O\_ISP(\lambda)$ that is a spectral reflectance characteristic of each of the objects (reference reflector plate 101F and inspection object 50) and then integrating the products on the wavelength axis.

First, the assumed light source L1 having a flat spectral characteristic is described with reference to graphs at the left side in D of FIG. 4 to G of FIG. 4.

D of FIG. 4 represents the spectral reflectance characteristic $O\_Ref(X)$ of the reference reflector plate 101F. Meanwhile, the reference reflector plate system overall spectral characteristic of E of FIG. 4 represents a characteristic obtained by multiplying the measurement system overall spectral characteristic $S(\lambda) \times I(\lambda)$ of C of FIG. 4 and the spectral reflectance characteristic $O\_Ref(\lambda)$ of the reference reflector plate 101F of D of FIG. 4. Further, the output value of the sensor 103 varies in proportion to the value obtained by integrating the characteristic of E of FIG. 4 on the wavelength axis (area of a region surrounded by a solid line representative of the light intensity of E of FIG. 4 and the wavelength axis).

It is to be noted that the intensity of the axis of ordinate of D of FIG. 4 represents the intensity of light reflected by the reference reflector plate 101F. Meanwhile, the intensity of the axis of ordinate of E of FIG. 4 represents an intensity obtained by multiplying the light intensity of C of FIG. 4 and the light intensity of D of FIG. 4.

Here, it is assumed that the R channel value and the IR channel value upon measurement of the reference reflector plate 101F under the assumed light source L1 are represented by $D\_Ref\_L1\_R = 1.0$ and $D\_Ref\_L1\_IR = 1.0$, respectively. In this case, the spectral ratio (R/IR ratio) of the reference reflector plate 101F under the assumed light source L1 is calculated by an expression (2) given below.

$$R\_Ref\_L1 = D\_Ref\_L1\_R/D\_Ref\_L1\_IR = 1.0/1.0 = 1.0$$

$$\cdots (2)$$

Further, F of FIG. 4 represents an example of a spectral reflectance characteristic $O\_Plt\_ISP(\lambda)$ of the inspection object 50. Further, the inspection object system overall spectral characteristic of G of FIG. 4 represents a characteristic obtained by multiplying the measurement system overall spectral characteristic $S(\lambda) \times I(\lambda)$ of C of FIG. 4 and the spectral reflectance characteristic $O\_Plt\_ISP(\lambda)$ of the inspection object 50 of F of FIG. 4. Then, the output value of the sensor 103 varies in proportion to a value obtained by integrating the characteristic of G of FIG. 4 on the wavelength axis (area of a region surrounded by a solid line representative of the light intensity of G of FIG. 4 and the wavelength axis).

It is to be noted that the intensity of the axis of ordinate in F of FIG. 4 represents the intensity of light reflected by the inspection object 50. Further, the intensity of the axis of ordinate in G of FIG. 4 represents the intensity obtained by multiplying the light intensity in C of FIG. 4 and the light intensity in F of FIG. 4.

Here, an R channel value and an IR channel value upon measurement of the inspection object 50 under the assumed light source L1 are determined as D_Plt_ISP_L1_R = 0.25 and D_Plt_ISP_L1_IR = 1.0, respectively. In this case, the spectral ratio (R/IR ratio) of the inspection object 50 under the assumed light source L1 is calculated by an expression (3) given below.

$$R\_Plt\_ISP\_L1 = D\_Plt\_ISP\_L1\_R/D\_Plt\_ISP\_L1\_IR = 0.25/1.0 = 0.25 ... \quad (3)$$

Then, measurement light correction for the spectral ratio (R/IR ratio) of the inspection object 50 determined by the expression (3) is represented by an expression (4) given below using the spectral ratio (R/IR ratio) of the reference reflector plate 101F determined by the expression (2). However, since the light source spectral characteristic of the assumed light source L1 in this case is flat, the measurement light correction is equivalent to that in an alternative case in which correction is not performed.

$$R\_Plt\_ISP\_L1\_comp = R\_Plt\_ISP\_L1/R\_Ref\_L1 = 0.25/1.0 = 0.25 ... \quad (4)$$

Now, an assumed light source L2 whose spectral characteristic is different from that of the assumed light source L1 and in which the intensity increases as the wavelength increases is described with reference to graphs at the right side in D of FIG. 4 to G of FIG. 4.

D of FIG. 4 represents a spectral reflectance characteristic O_Ref(λ) of the reference reflector plate 101F. Further, a reference reflector plate system overall spectral characteristic of E of FIG. 4 represents a characteristic obtained by multiplying the measurement system overall spectral characteristic S(λ) × I(λ) of C of FIG. 4 and the spectral reflectance characteristic O_Ref(λ) of the reference reflector plate 101F of D of FIG. 4.

Here, the R channel value and the IR channel value upon measurement of the reference reflector plate 101 under the assumed light source L2 are determined as D_Ref_L2_R = 0.25 and D_Ref_L2_IR = 1.0, respectively. In this case, the spectral ratio (R/IR ratio) of the reference reflector plate 101F under the assumed light source L2 is calculated by an expression (5) given below.

$$R\_Ref\_L2 = D\_Ref\_L2\_R/D\_Ref\_L2\_IR = 0.25/1.0 = 0.25 ... \quad (5)$$

Further, F of FIG. 4 represents an example of the spectral reflectance characteristic O_Plt_ISP(λ) of the inspection object 50. Further, the inspection object system overall spectral characteristic of G of FIG. 4 represents a characteristic obtained by multiplying the measurement system overall spectral characteristic S(λ) × I(λ) of C of FIG. 4 and the spectral reflectance characteristic O_Plt_ISP(λ) of the inspection object 50 of F of FIG. 4.

Here, the R channel value and the IR channel value upon measurement of the inspection object 50 under the assumed light source L2 are determined as D_Plt_ISP_L2_R = 0.05 and D_Plt_ISP_L2_IR = 1.0, respectively. In this case, the spectral ratio (R/IR ratio) of the inspection object 50 under the assumed light source L2 is calculated by an expression (6) given below.

$$R\_Plt\_ISP\_L2 = D\_Plt\_ISP\_L2\_R/D\_Plt\_ISP\_L2\_IR = 0.05/1.0 = 0.05 ... \quad (6)$$

Then, measurement light correction for the spectral ratio (R/IR ratio) of the inspection object 50 calculated by the expression (6) is represented by an expression (7) given below using the spectral ratio (R/IR ratio) of the reference reflector plate 101F determined by the expression (5).

$$R\_Plt\_ISP\_L2\_comp = R\_Plt\_ISP\_L2/R\_Ref\_L2 = 0.05/0.25 = 0.20 ... \quad (7)$$

Measurement light correction is performed in such a manner as described above. However, although, from a point of view of the correction accuracy in measurement light correction for the spectral ratio (R/IR ratio) of the inspection object 50, the measurement value under the assumed light source L2 (R_Plt_ISP_L2 = 0.05) is much different from the measurement value under the assumed light source L1 (R_Plt_ISP_L1 = 0.25), it is corrected to a closer value (R_Plt_ISP_L1_comp = 0.25, R_ISP_L2_comp = 0.20) by the measurement light correction described hereinabove.

However, even where the measurement light correction is performed, there still remains an error.

This arises from the fact that, while the correction gain to the spectral ratio (R/IR ratio) corresponding to the output value of the sensor 103 for the spectral characteristic of G of FIG. 4 is determined from the spectral ratio (R/IR ratio) corresponding to the output value of the sensor 103 for the spectral characteristic of E of FIG. 4, since a great difference exists in the components (O_Ref($\lambda$) and O_Plt_ISP($\lambda$)) of the spectral reflectance of the object between G of FIG. 4 and E of FIG. 4, the applied correction gain is not a correction gain having a high degree of accuracy with respect to the inspection object 50.

(Case in which measurement light correction is performed using lookup table (LUT))

FIG. 5 is a view illustrating a case in which measurement light correction is performed using a lookup table (LUT). It is to be noted that, in FIG. 5, the axes of the graphs are similar to those of the graphs of FIG. 4.

Since a sensor spectral sensitivity characteristic S($\lambda$) of A of FIG. 5, a light source spectral characteristic I($\lambda$) of B of FIG. 5 and a measurement system overall spectral characteristic S($\lambda$) $\times$ I($\lambda$) of C of FIG. 5 are similar to the sensor spectral sensitivity characteristic S($\lambda$) of A of FIG. 4, light source spectral characteristic I($\lambda$) of B of FIG. 4 and measurement system overall spectral characteristic S($\lambda$) $\times$ I($\lambda$) of C of FIG. 4, respectively, description of them is omitted.

First, a creation method of a lookup table (LUT) is described with reference to D of FIG. 5 and E of FIG. 5 as well as H of FIG. 5 and I of FIG. 5.

D of FIG. 5 represents a spectral reflectance characteristic O_Ref($\lambda$) of the reference reflector plate 101, and the value is flat. Further, a reference reflector plate system overall spectral characteristic of E of FIG. 5 represents a characteristic obtained by multiplying the measurement system overall spectral characteristic S($\lambda$) $\times$ I($\lambda$) of C of FIG. 5 and the spectral reflectance characteristic O_Ref($\lambda$) of the reference reflector plate 101 of D of FIG. 5.

Here, the R channel value and the IR channel value upon measurement of the reference reflector plate 101 under the assumed light source L1 and the assumed light source L2 are determined as D_Ref_L1_R = 1.0, D_Ref_L1_IR = 1.0, and D_Ref_L2_R = 0.25, D_Ref_L2_IR = 1.0. In this case, the spectral ratios (R/IR ratio) of the reference reflector plate 101 under the assumed light source L1 and assumed light source L2 are calculated by expressions (8) and (9) given below, respectively.

$$\text{R\_Ref\_L1 = D\_Ref\_L1\_R/D\_Ref\_L1\_IR = 1.0/1.0 = 1.0}$$
$$\ldots (8)$$

$$\text{R\_Ref\_L2 = D\_Ref\_L2\_R/D\_Ref\_L2\_IR = 0.25/1.0 = 0.25}$$
$$\ldots (9)$$

H of FIG. 5 represents a spectral reflectance representative characteristic O_Plt_AVE($\lambda$) of the inspection object 50. The spectral reflectance representative characteristic is a representative characteristic of the inspection object 50 whose characteristic varies. Here, while a case in which an average value of a plurality of spectral reflectances is adopted is described as the representative characteristic, some other characteristic may be used. Further, where an average value is adopted as the representative characteristic, although the spectral reflectance representative characteristic O_Plt_AVE($\lambda$) and spectral reflectance characteristics O_Plt_ISP($\lambda$) of individual inspection objects 50 do not necessarily coincide with each other, here a case in which the characteristics coincide with each other is described as the most effective example.

An inspection object system overall spectrum representative characteristic of I of FIG. 5 represents a characteristic obtained by multiplying the measurement system overall spectral characteristic S($\lambda$) $\times$ I($\lambda$) of C of FIG. 5 and the spectral reflectance representative characteristic O_Plt_AVE($\lambda$) of H of FIG. 5. Further, the output value of the sensor 103 varies in proportion to a value obtained by integrating the characteristic of I of FIG. 5 on the wavelength axis (area of a region surrounded by a solid line representative of a light intensity and a wavelength axis in I of FIG. 5).

It is to be noted that the intensity of the axis of ordinate of H of FIG. 5 represents the intensity of light reflected by the inspection object 50. Further, the intensity of the axis of ordinate of I of FIG. 5 represents the intensity obtained by multiplying the light intensity of C of FIG. 5 and the light intensity of H of FIG. 5.

Here, assuming a case in which the inspection object 50 having the spectral reflectance representative characteristic O_Plt_AVE($\lambda$) is measured under the assumed light source L1 and assumed light source L2, the R channel value and the IR channel value of the light sources are determined as D_Plt_AVE_L1_R = 0.25, D_Plt_AVE_L1_IR = 1.0, and D_Plt_AVE_L2_R = 0.05, D_Plt_AVE_L2_IR = 1.0, respectively. In this case, the spectral ratios (R/IR ratio) of the inspection object 50 under the assumed light source L1 and the assumed light source L2 are calculated by expressions (10) and (11) given below, respectively.

```
R_Plt_AVE_L1 = D_Plt_AVE_L1_R/D_Plt_AVE_L1_IR = 0.25/1.0 = 0.25 ...
(10)
```

```
R_Plt_AVE_L2 = D_Plt_AVE_L2_R/D_Plt_AVE_L2_IR = 0.05/1.0 = 0.05 ...
(11)
```

Further, correction gains for the assumed light source L1 and the assumed light source L2 are calculated by expressions (12) and (13) given below. However, the assumed light source L1 here is a reference light source.

```
G_Plt_AVE_L1 = R_Plt_AVE_L1/R_Plt_AVE_L1 = 0.25/0.25 = 1.0 ... (12)
```

```
G_Plt_AVE_L2 = R_Plt_AVE_L1/R_Plt_AVE_L2 = 0.25/0.05 = 5.0 ... (13)
```

Further, since the spectral ratio (R/IR ratio) of the reference reflector plate 101 and the correction gain are calculated for each assumed light source, information of them is stored into the lookup table (LUT). In particular, with the assumed light source L1, R_Ref_L1 = 1.0 calculated by the expression (8) and G_Plt_AVE_L1 = 1.0 calculated by the expression (12) are associated, and with the assumed light source L2, R_Ref_L2 = 0.25 calculated by the expression (9) and G_Plt_AVE_L2 = 5.0 calculated by the expression (13) are associated.

Lookup table (LUT):

    Assumed light source L1: R Ref L1 (= 1.0): G_Plt_AVE_L1 (= 1.0)
    Assumed light source L2: R_Ref_L2 (= 0.25): G_Plt_AVE_L2 (= 5.0)

The lookup table (LUT) can be created in such a manner as described above.
Now, measurement of the R/IR ratio by measurement of the inspection object 50 under various measurement light sources and measurement light correction for the spectral ratio (R/IR ratio) of the inspection object 50 in which the lookup table (LUT) described above is used are described with reference to F of FIG. 5 and G of FIG. 5.
F of FIG. 5 represents an example of the spectral reflectance characteristic O_Plt_ISP($\lambda$) of the inspection object 50, and here, it is assumed that the spectral reflectance characteristic O_Plt_ISP($\lambda$) coincides with the spectral reflectance representative characteristic O_Plt_AVE($\lambda$) of the inspection object 50 illustrated in H of FIG. 5. Further, the inspection object system overall spectral characteristic of G of FIG. 5 represents a characteristic obtained by multiplying the measurement system overall spectral characteristic $S(\lambda) \times I(\lambda)$ of C of FIG. 5 and the spectral reflectance characteristic O_Plt_ISP($\lambda$) of the inspection object 50 of F of FIG. 5.
Here, where the inspection object 50 having the spectral reflectance characteristic O_Plt_ISP($\lambda$) is measured under the measurement light source L1 and the measurement light source L2, the R channel value and the IR channel value for each light source are determined as D_Plt_ISP_L1_R = 0.25, D_Plt_ISP_L1_IR = 1.0, D_Plt_ISP_L2_R = 0.05 and D_Plt_ISP_L2_IR = 1.0. In this case, the spectral ratios (R/IR ratios) of the inspection object 50 under the measurement light source L1 and the measurement light source L2 are calculated by expressions (14) and (15) given below, respectively.

```
R_Plt_ISP_L1 = D_Plt_ISP_L1_R/D_Plt_ISP_L1_IR = 0.25/1.0 = 0.25 ...
(14)
```

```
R_Plt_ISP_L2 = D_Plt_ISP_L2_R/D_Plt_ISP_L2_IR = 0.05/1.0 = 0.05 ...
(15)
```

Here, where it is assumed that the measurement light source L1 and the assumed light source L1 coincide with each other, the spectral ratio (R/IR ratio) of the reference reflector plate 101 under the measurement light source L1 is R_Ref_L1 = 1.0, and therefore, referring to the lookup table (LUT), it is determined that the assumed light source L1 is an estimated light source. Consequently, G_Plt_AVE_L1 = 1.0 is determined as a correction gain according to the spectral ratio (R_Ref_L1 = 1.0) of the reference reflector plate 101 under the measurement light source L1 on the basis of the lookup table (LUT).

Then, using the correction gain determined in such a manner as described above, the spectral ratio (R/IR ratio) of the inspection object 50 under the measurement light source L1 is corrected in accordance with an expression (16) given below.

```
R_Plt_ISP_L1_comp = R_Plt_ISP_L1 × G_Plt_AVE_L1 = 0.25 × 1.0 = 0.25 ...
(16)
```

On the other hand, where it is assumed that the measurement light source L2 coincides with the assumed light source L2, since the spectral ratio (R/IR ratio) of the reference reflector plate 101 under the measurement light source L2 becomes R_Ref_L2 = 0.25, it is determined referring to the lookup table (LUT) that the assumed light source L2 is an estimated light source. Consequently, G_Plt_AVE_L2 = 5.0 is determined as a correction gain according to the spectral ratio (R_Ref_L2 = 0.25) of the reference reflector plate 101 under the measurement light source L2 on the basis of the lookup table (LUT).

Then, the spectral ratio (R/IR ratio) of the inspection object 50 under the measurement light source L2 is corrected in accordance with an expression (17) given below using the correction gain determined in such a manner as described above.

```
R_Plt_ISP_L2_comp = R_Plt_ISP_L2 × G_Plt_AVE_L2 = 0.05 × 5.0 = 0.25 ...
(17)
```

While measurement light correction is performed in such a manner as described, from a point of view of the correction accuracy in measurement light correction for the spectral ratio (R/IR ratio) of the inspection object 50, the measurement value (R_Plt_ISP_L2 = 0.05) under the measurement light source L2 is much different from the measurement value (R_Plt_ISP_L1 = 0.25) under the measurement light source L1. However, the measurement values are corrected to equal values (R_Plt_ISP_L1_comp = R_Plt_ISP_L2_comp (= 0.25)) by the measurement light correction to which the expression (17) is applied.

In regard to a reason therefor, this arises from the fact that the correction gain to the spectral ratio (R/IR ratio) corresponding to the output of the sensor 103 for the spectral characteristic of G of FIG. 5 is not determined directly from the spectral ratio (R/IR ratio) corresponding to the output of the sensor 103 for the spectral characteristic of E of FIG. 5 but is determined using the inspection object system overall spectral representative characteristic of I of FIG. 5, and in this case, between I of FIG. 5 and G of FIG. 5, there is a very high correlation between the components (O_Plt_AVE(λ) and O_Plt_ISP(λ)) of the spectral reflectance of the object that are one of factors that create them, characteristic and so forth. Therefore, the reason mentioned above arises from that the applied correction gain can become a correction gain having a high degree of accuracy to the inspection object 50.

It is to be noted that, while a case is described in which the spectral reflectance representative characteristic O_Plt_AVE(λ) of the inspection object 50 and the spectral reflectance characteristic O_Plt_ISP(λ) of the inspection object 50 that are used upon creation of a lookup table (LUT) are equal to each other in FIG. 5, even if the characteristics are not fully equal to each other, since the two characteristics have a very high correlation, the error in measurement light correction can be minimized in comparison with the conventional technology.

<3. Creation of Lookup Table>

Now, a creation method of a lookup table (LUT) to be used by the vegetation inspection apparatus 10 (FIG. 1) is described.

(Configuration of lookup table creation apparatus)

FIG. 6 is a view depicting an example of configuration of a lookup table creation apparatus 20.

The lookup table creation apparatus 20 of FIG. 6 is an apparatus for creating (generating) a lookup table (LUT) to be used by the vegetation inspection apparatus 10 (FIG. 1).

In FIG. 6, the lookup table creation apparatus 20 is configured from an inspection object average spectral characteristic calculation unit 201, a reference reflector plate measurement sensor output value calculation unit 202, a reference reflector plate R/IR ratio calculation unit 203, an inspection object measurement sensor output value calculation unit 204, an inspection object R/IR ratio calculation unit 205, a correction gain calculation unit 206 and a lookup table creation unit 207.

It is to be noted that, in FIG. 6, the measurement apparatus 21 is provided at the preceding stage to the lookup table creation apparatus 20, and a measurement value of an object measured by the measurement apparatus 21 is inputted to the lookup table creation apparatus 20. Further, in FIG. 6, a case in which light sources having color temperatures of 2800k, 5500k and 10000k are determined as assumed light sources and the light source having a color temperature of 5500k from among the assumed light sources is determined as a light source having a reference color temperature (reference light source) is described as an example.

A measurement value of the spectral reflectance $O\_Plt\_i(\lambda)$ of the inspection object 50 is inputted from the measurement apparatus 21 to the inspection object average spectral characteristic calculation unit 201. In the spectral reflectance $O\_Plt\_i(\lambda)$, i is an integer equal to or greater than 1 (i = 1, ..., N) and is an index indicative of a number of an individual having a different characteristic among individuals included in the inspection object 50. Here, the characteristic can be a vegetation representing an amount or activity of a plant with respect to a same kind of plants.

The inspection object average spectral characteristic calculation unit 201 calculates an average spectral reflectance characteristic $O\_Plt\_i(\lambda)$ of N spectral reflectance characteristics $O\_Plt\_i(\lambda)$ on the basis of the spectral reflectance characteristic $O\_Plt\_i(\lambda)$ of the inspection object 50 and supplies the average spectral reflectance characteristic $O\_Plt\_i(\lambda)$ to the inspection object measurement sensor output value calculation unit 204.

To the reference reflector plate measurement sensor output value calculation unit 202, known spectral information of the assumed light sources $(I\_2800k(\lambda), I\_5500k(\lambda), I\_10000k(X))$, known spectral sensitivity characteristic $(S\_R(\lambda), S\_IR(\lambda))$ of the channels (of the sensor) of the measurement apparatus 21 and a measurement value of the spectral reflectance characteristic $O\_Ref\_i(\lambda)$ of the reference reflector plate 101 are inputted from the measurement apparatus 21.

The reference reflector plate measurement sensor output value calculation unit 202 calculates an R channel value and an IR channel value in the case in which the reference reflector plate 101 is measured under the assumed light sources of the color temperatures on the basis of the known spectral information $(I\_2800K(\lambda), I\_5500K(\lambda), I\_10000K(\lambda))$ of the assumed light sources, known spectral sensitivity characteristics $(S\_R(\lambda), S\_IR(\lambda))$ of the channels and the spectral reflectance characteristic $O\_Ref(\lambda)$ of the reference reflector plate 101.

Here, channel values $(D\_Ref\_2800K\_R, D\_Ref\_2800K\_IR)$ under the assumed light source having the color temperature of 2800K, channel values $(D\_Ref\_5500K\_R, D\_Ref\_5500K\_IR)$ under the assumed light source having the color temperature of 5500K and channel values $(D\_Ref\_10000K\_R, D\_Ref\_10000K\_IR)$ under the assumed light source having the color temperature of 10000K are calculated and supplied to the reference reflector plate R/IR ratio calculation unit 203.

The reference reflector plate R/IR ratio calculation unit 203 calculates the assumed spectral ratios (R/IR ratios) of the reference reflector plate 101 under the assumed light sources having the color temperatures on the basis of the R channel values and the IR channel values obtained where the reference reflector plate 101 is measured under the assumed light source of the color temperatures and supplied from the reference reflector plate measurement sensor output value calculation unit 202.

Here, the assumed spectral ratios (D Ref 2800K, D Ref 5500K, D_Ref_10000K) of the reference reflector plate 101 are calculated on the basis of the channel values $(D\_Ref\_2800K\_R$ and $D\_Ref\_2800K\_IR, D\_Ref\_5500K\_R$ and $D\_Ref\_5500K\_IR$, and $D\_Ref\_10000K\_R$ and $D\_Ref\_10000K\_IR)$ under the assumed light sources having the color temperatures of 2800K, 5500K and 10000K, and are supplied to the lookup table creation unit 207.

Further, to the inspection object measurement sensor output value calculation unit 204, the known spectral information $(I\_2800K(\lambda), I\_5500K(\lambda), I\_10000K(\lambda))$ of the assumed light sources, known spectral sensitivity characteristics $(S\_R(\lambda), S\_IR(\lambda))$ of the channels of (the sensor of) the measurement apparatus 21 and average reflectance $O\_Plt\_AVE(\lambda)$ of the inspection object 50 supplied from the inspection object average spectral characteristic calculation unit 201 are inputted.

The inspection object measurement sensor output value calculation unit 204 calculates an R channel value and an IR channel where the inspection object 50 is measured under the assumed light sources of the color temperatures on the base of the known spectral information $(I\_2800K(\lambda), I\_5500K(\lambda), I\_10000K(\lambda))$ of the assumed light sources, known spectral sensitivity characteristics $(S\_R(\lambda), S\_IR(\lambda))$ of the channels and average reflectance $O\_Plt\_AVE(\lambda)$ of the inspection object 50.

Here, channel values $(D\_Plt\_AVE\_2800K\_R, D\_Plt\_AVE\_2800K\_IR)$ under the assumed light source of the color temperature of 2800K, channel values $(D\_Plt\_AVE\_5500K\_R, D\_Plt\_AVE\_5500K\_IR)$ under the assumed light source of the color temperature of 5500K and channel values $(D\_Plt\_AVE\_10000K\_R, D\_Plt\_AVE\_10000K\_IR)$ under the assumed light source of the color temperature of 10000K are calculated and supplied to the inspection object R/IR ratio calculation unit 205. However, the channel values under the measurement light sources of the color temperatures are determined as channel values of a representative value such as an average value.

The inspection object R/IR ratio calculation unit 205 calculates an assumed spectral ratio (R/IR ratio) of the inspection object 50 under the assumed light source of each color temperature on the basis of the R channel values and the IR channel values obtained where the inspection object 50 is measured under the assumed light sources of the color temperatures and supplied from the inspection object measurement sensor output value calculation unit 204.

Here, assumed spectral ratios (R_Plt_AVE_2800K, R_Plt_AVE_5500K, R_Plt_AVE_10000K) of the inspection object 50 are calculated on the basis of the channel values (D_Plt_AVE_2800K_R, D_Plt_AVE_2800K_IR, D_Plt_AVE_5500K_R, D_Plt_AVE_5500K_IR, D_Plt_AVE_10000K_R, D_Plt_AVE_10000K_IR) under the assumed light sources of the color temperatures of 2800K, 5500K and 10000K, and are supplied to the correction gain calculation unit 206. However, the assumed spectral ratio (R/IR ratio) of the inspection object 50 is determined as an assumed spectral ratio (R/IR ratio) of the representative value such as an average value.

The correction gain calculation unit 206 calculates a correction gain for correcting the measurement spectral ratios (R/IR ratios) of the inspection object 50 obtained by measurement (sensing) under the measurement light source on the basis of the assumed spectral ratios (R/IR ratios) of the inspection object 50 under the assumed light sources of the color temperatures supplied from the inspection object R/IR ratio calculation unit 205.

Here, since the assumed light source of the color temperature of 5500K from among the assumed light sources of the color temperatures of 2800K, 5500K and 10000K is determined as a light source of the reference color temperature (reference light source), the correction gain is a correction gain for correcting the measurement spectral ratio (R/IR value of the representative value) of the inspection object 50 calculated when the inspection object 50 is measured (sensed) under the assumed light sources of the color temperatures so as to be equal to the measurement spectral ratio (R/IR ratio of the representative value) calculated when the inspection object 50 is measured under the light source (reference light source) of the reference color temperature of 5500K.

Then, correction gains (G_Plt_AVE_2800K, G_Plt_AVE_5500K, G_Plt_AVE_10000K) under the assumed light sources of the color temperatures according to the assumed spectral ratios (R_Plt_AVE_2800K, R_Plt_AVE_5500K, R_Plt_AVE_10000K) of the inspection object 50 under the assumed light sources of the color temperature of 2800K, 5500K and 10000K are calculated and supplied to the lookup table creation unit 207.

To the lookup table creation unit 207, the assumed spectral ratios (R_Ref_2800K, R_Ref_5500K, R_Ref_10000K) of the reference reflector plate 101 from the reference reflector plate R/IR ratio calculation unit 203 and the correction gains (G_Plt_AVE_2800K, G_Plt_AVE_5500K, G_Plt_AVE_10000K) from the correction gain calculation unit 206 are supplied.

The lookup table creation unit 207 creates (generates) a lookup table (LUT) on the basis of the assumed spectral ratios (R_Ref_2800K, R_Ref_5500K, R_Ref_10000K) of the reference reflector plate 101 under the assumed light sources of the color temperatures and the correction gains (G_Plt_AVE_2800K, G_Plt_AVE_5500K, G_Plt_AVE_10000K) under the assumed light sources of the color temperatures.

In the lookup table (LUT) created in this manner, the reference spectral ratios (R_Ref_2800K, R_Ref_5500K, R_Ref_10000K) (of the reference reflector plate 101) and the correction gains (G_Plt_AVE_2800K, G_Plt_AVE_5500K, G_Plt_AVE_10000K) for correcting the measurement spectral ratios (R/IR ratios) of the inspection object 50 obtained by the measurement (sensing) under the measurement light sources are associated with each other for the individual assumed light sources of the color temperatures (2800K, 5500K, 10000K). However, the reference spectral ratios (R_Ref_2800K, R_Ref_5500K, R_Ref_10000K) are spectral ratios referred to upon measurement of the inspection index (NDVI value) of the inspection object 50 under the measurement light sources and correspond to the assumed spectral ratios (R_Ref_2800K, R_Ref_5500K, R_Ref_10000K) of the reference reflector plate 101.

It is to be noted that, while the lookup table creation apparatus 20 and the measurement apparatus 21 in FIG. 6 are described as separate apparatus for the convenience of description, the measurement apparatus 21 may otherwise be included in the lookup table creation apparatus 20. Further, parameters such as known spectral information of the assumed light sources inputted from the measurement apparatus 21 to the lookup table creation apparatus 20 may otherwise be inputted from an external apparatus.

(Lookup table creation process)

Now, a creation process of a lookup table (LUT) executed by the lookup table creation apparatus 20 and the measurement apparatus 21 of FIG. 6 and used by the vegetation inspection apparatus 10 (FIG. 1) is described with reference to a flow chart of FIG. 7.

Here, before the process of the flow chart of FIG. 7 is executed, an assumed light source and a reference light source are determined, for example, by the user or in accordance with a value prescribed in advance. For example, as depicted in FIG. 8, light sources of the color temperatures of 2800K, 5500K and 10000K are determined as assumed light sources, and further, from among the assumed light sources, the light source of the color temperature of 5500K is determined as a light source of a reference color temperature (reference light source).

Referring back to FIG. 7, at step S11, the measurement apparatus 21 measures or calculates known spectral information of the assumed light sources. Here, as the known spectral information of the assumed light sources of the color temperatures of 2800K, 5500K and 10000K, I_2800K(λ), I_5500K(λ), I_10000K(λ) are measured or calculated.

At step S12, the measurement apparatus 21 measures a known spectral sensitivity characteristic of each channel of the sensor. Here, S_R(λ) is measured as the known spectral sensitivity characteristic of the R channel, and S_IR(λ) is measured as the known spectral sensitivity characteristic of the IR channel.

At step S13, the measurement apparatus 21 measures a spectral reflectance characteristic O_Ref(λ) of an arbitrary reference reflector plate 101.

At step S14, the measurement apparatus 21 measures spectral reflectance characteristics O_Plt_i(λ) (i = 1, ..., N) of a plurality of inspection objects 50 of characteristics different from each other. In the spectral reflectances O_Plt_i(λ), i is an integer equal to or greater than 1 (i = 1, ..., N) and is an index indicative of a number of an individual different in characteristic among the individuals included in the inspection object 50.

In this manner, at steps S11 to S14, as a preprocess (preparation process) for a creation process of a lookup table (LUT), the known spectral information (I_2800K(λ), I_5500K(λ), I_10000K(λ)) of the assumed light sources, known spectral sensitivity characteristics (S_R(λ), S_IR(λ)) of the channels of the sensor, spectral reflectance characteristic O_Ref(λ) of the reference reflector plate 101 and spectral reflectance characteristic O_Plt_i(λ) of the inspection object 50 are measured by the measurement apparatus 21 and are outputted to the lookup table creation apparatus 20.

At step S15, the inspection object average spectral characteristic calculation unit 201 arithmetically operates an expression (18) given below on the basis of the spectral reflectance characteristic O_Plt_i(λ) of the inspection object 50 measured by the process at step S14 to calculate the average spectral reflectance characteristic O_Plt_AVE(λ) of the N spectral reflectance characteristics O_Plt_i(λ).

[Math. 1]

$$O\_Plt\_AVE(\lambda) = 1/N \sum_{i=1}^{N} O\_Plt\_i(\lambda) \ \ldots \ (18)$$

At step S16, the reference reflector plate measurement sensor output value calculation unit 202 arithmetically operates expressions (19) to (24) given below on the basis of the spectral information (I_2800K(λ), I_5500K(λ), I_10000K(λ)) of the assumed light sources measured by the process at step S11, spectral sensitivity characteristics (S_R(λ), S_IR(λ)) of the channels measured by the process at step S12, and spectral reflectance characteristic O_Ref(λ) of the reference reflector plate 101 measured by the process at step S13 to calculate an R channel value and an IR channel value in the case where the reference reflector plate 101 is measured (sensed) under the assumed light source of each color temperature.

However, by expressions (19) to (21) given below, R channel values according to the assumed light sources of the color temperatures (2800K, 5500K, 10000K) are calculated, and by expressions (22) to (24) given below, IR channel values according to the assumed light sources of the color temperatures (2800K, 5500K and 10000K) are calculated.

[Math. 2]

$$D\_Ref\_2800K\_R = \int I\_2800K(\lambda)O\_Ref(\lambda)S\_R(\lambda)d\lambda \ \ldots \ (19)$$

$$D\_Ref\_5500K\_R = \int I\_5500K(\lambda)O\_Ref(\lambda)S\_R(\lambda)d\lambda \ \ldots \ (20)$$

$$D\_Ref\_10000K\_R = \int I\_10000K(\lambda)O\_Ref(\lambda)S\_R(\lambda)d\lambda \ \ldots \ (21)$$

[Math. 3]

$$D\_Ref\_2800K\_IR = \int I\_2800K(\lambda)O\_Ref(\lambda)S\_IR(\lambda)d\lambda \ \ldots \ (22)$$

$$D\_Ref\_5500K\_IR = \int I\_5500K(\lambda)O\_Ref(\lambda)S\_IR(\lambda)d\lambda \ \ldots \ (23)$$

$$D\_Ref\_10000K\_IR = \int I\_10000K(\lambda)O\_Ref(\lambda)S\_IR(\lambda)d\lambda \ \ldots \ (24)$$

At step S17, the reference reflector plate R/IR ratio calculation unit 203 arithmetically operates expressions (25) to (27) given below on the basis of the R channel values and the IR channel values calculated by the process at step S16 to calculate assumed spectral ratios (R/IR ratios) of the reference reflector plate 101 under the assumed light source of each color temperature.

[Math. 4]

$$R\_Ref\_2800K = D\_Ref\_2800K\_R/D\_Ref\_2800K\_IR \ldots (25)$$

$$R\_Ref\_5500K = D\_Ref\_5500K\_R/D\_Ref\_5500K\_IR \ldots (26)$$

$$R\_Ref\_10000K = D\_Ref10000K\_R/D\_Ref\_10000K\_IR \ldots (27)$$

At step S18, the inspection object measurement sensor output value calculation unit 204 arithmetically operates expressions (28) to (33) given below on the basis of the spectral information (I_2800K($\lambda$), I_5500K($\lambda$), I_10000K($\lambda$)) of the assumed light sources measured by the process at step S11, spectral sensitivity characteristics (S_R($\lambda$), S_IR($\lambda$)) of the channels measured by the process at step S12, and average spectral reflectance characteristic O_Plt_i($\lambda$) of the inspection object 50 calculated by the process at step S15 to calculate R channel values and IR channel values when the inspection object 50 is measured (sensed) under the assumed light source of each color temperature.

However, by the expressions (28) to (30) given below, R channel values according to the assumed light sources of the color temperatures (2800K, 5500K, 10000K), and by the expressions (31) to (33) given below, IR channel values according to the assumed light sources of the color temperatures (2800K, 5500K, 10000K) are calculated. Further, the channel values under the measurement light sources of the color temperatures are channel values of a representative value such as an average value.

[Math. 5]

$$D\_Plt\_AVE\_2800K\_R = \int I\_2800K(\lambda)O\_Plt\_AVE(\lambda)S\_R(\lambda)d\lambda \ldots (28)$$

$$D\_Plt\_AVE\_5500K\_R = \int I\_5500K(\lambda)O\_Plt\_AVE(\lambda)S\_R(\lambda)d\lambda \ldots (29)$$

$$D\_Plt\_AVE\_10000K\_R = \int I\_10000K(\lambda)O\_Plt\_AVE(\lambda)S\_R(\lambda)d\lambda \ldots (30)$$

[Math. 6]

$$D\_Plt\_AVE\_2800K\_IR = \int I\_2800K(\lambda)O\_Plt\_AVE(\lambda)S\_IR(\lambda)d\lambda \ldots (31)$$

$$D\_Plt\_AVE\_5500K\_IR = \int I\_5500K(\lambda)O\_Plt\_AVE(\lambda)S\_IR(\lambda)d\lambda \ldots (32)$$

$$D\_Plt\_AVE\_10000K\_IR = \int I\_10000K(\lambda)O\_Plt\_AVE(\lambda)S\_IR(\lambda)d\lambda \ldots (33)$$

At step S19, the inspection object R/IR ratio calculation unit 205 arithmetically operates expressions (34) to (36) given below on the basis of the R channel values and the IR channel values calculated by the process at step S18 to calculate assumed spectral ratios (R/IR ratios) of the inspection object 50 under the assumed light sources of the color temperatures. However, the assumed spectral radios (R/IR ratios) of the inspection object 50 are assumed spectral ratios (R/IR ratios) of a representative value such as an average value.

[Math. 7]

$$R\_Plt\_AVE\_2800K = D\_Plt\_AVE\_2800K\_R/D\_Plt\_AVE\_2800K\_IR$$

$$\dots (34)$$

$$R\_Plt\_AVE\_5500K = D\_Plt\_AVE\_5500K\_R/D\_Plt\_AVE\_5500K\_IR$$

$$\dots (35)$$

$$R\_Plt\_AVE\_10000K = D\_Plt\_AVE\_10000K\_R/D\_Plt\_AVE\_10000K\_IR \dots (36)$$

At step S20, the correction gain calculation unit 206 arithmetically operates expressions (37) to (39) given below on the basis of the assumed spectral ratios (R/IR ratios) of the inspection object 50 under the assumed light sources of the color temperatures calculated by the process at step S19 to calculate correction gains for correcting the measurement spectral ratios (R/IR ratios) of the inspection object 50 for the individual assumed light sources.
[Math. 8]

$$G\_Plt\_AVE\_2800K = R\_Plt\_AVE\_5500K/R\_Plt\_AVE\_2800K$$

$$\dots (37)$$

$$G\_Plt\_AVE\_5500K = R\_Plt\_AVE\_5500K/R\_Plt\_AVE\_5500K = 1$$

$$\dots (38)$$

$$G\_Plt\_AVE\_10000K = R\_Plt\_AVE\_5500K/R\_Plt\_AVE\_10000K$$

$$\dots (39)$$

At step S21, the lookup table creation unit 207 creates a lookup table (LUT) on the basis of the assumed spectral ratios (R_Ref_2800K, R_Ref_5500K, R_Ref_10000K) of the reference reflector plate 101 calculated by the process at step S17 and the correction gains (G_Plt_AVE_2800K, G_Plt_AVE_5500K, G_Plt_AVE_10000K) calculated by the process at step S20.
In this lookup table (LUT), with the individual assumed light sources of the color temperatures (2800K, 5500K, 10000K), the reference spectral ratios (R_Ref_2800K, R_Ref_5500K, R_Ref_10000K) (of the reference reflector plate 101) and the correction gains (G_Plt_AVE_2800K, G_Plt_AVE_5500K, G_Plt_AVE_10000K) for correcting the measurement spectral ratios (R/IR ratios) of the inspection object 50 are associated. However, in the lookup table (LUT), the reference spectral ratios correspond to the assumed spectral ratios (R_Ref_2800K, R_Ref_5500K, R_Ref_10000K) of the reference reflector plate 101.
In particular, as depicted by the example of the lookup table (LUT) of FIG. 9, R_Ref_2800K and G_Plt_AVE_2800K are associated with the assumed light source of the color temperature of 2800K; R_Ref_5500K and G_Plt_AVE_5500K are associated with the assumed light source of the color temperature of 5500K; and R_Ref_10000K and G_Plt_AVE_10000K are associated with the assumed light source of the color temperature of 10000K.
When the process at step S21 ends, the lookup table creation process of FIG. 7 is ended.
The lookup table creation process has been described. In this lookup table creation process, assumed spectral ratios (R_Ref_2800K, R_Ref_5500K, R_Ref_10000K) of the reference reflector plate 101 and assumed spectral ratios (R_Plt_AVE_2800K, R_Plt_AVE_5500K, R_Plt_AVE_10000K) of the inspection object 50 under assumed light sources of a plurality of color temperatures are calculated, and a lookup table (LUT) that associates reference spectral ratios of the reference reflector plate 101 and correction gains according to assumed spectral ratios of the inspection object 50 with each other is created for each assumed light source. Then, the lookup tables (LUTs) created in this manner are used by the vegetation inspection apparatus 10 (FIG. 1) (for example, are stored into a storage unit 112 of a correction processing unit 105 of FIG. 10 or FIG. 13 hereinafter described).
It is to be noted that the lookup table creation process described above is an example of a method for creating a lookup table (LUT) to be used by the vegetation inspection apparatus 10 (FIG. 1), and any other creation method may be adopted if it is a method that can create a lookup table (LUT) such as, for example, to use a different characteristic in place of the average spectral reflectance characteristic O_Plt_AVE($\lambda$) of the inspection object 50.

<4. Inspection of Inspection Object>

Now, an inspection method of the inspection object 50 performed by the vegetation inspection apparatus 10 (FIG. 1) that uses a lookup table (LUT) created by the creation method described above is described. Here, when an arithmetic operation for calculating an inspection index (NDVI value) is executed, a configuration and a process where normalization of values to be used in the arithmetic operation is not performed (FIGS. 10 to 12) are described first, and then a configuration and a process where normalization of values to be used in the arithmetic operation is performed (FIGS. 13 and 14) is described.

(First functional configuration of vegetation inspection apparatus)

FIG. 10 is a view depicting a first functional configuration of the vegetation inspection apparatus 10 (FIG. 1) upon measurement of an inspection index (NDVI value) of the inspection object 50 under a measurement light source.

It is to be noted that, in FIG. 10, only a sensor 103, a correction processing unit 105 and an inspection index calculation unit 106 that are blocks especially relating to measurement of an inspection index of the inspection object 50 under a measurement light source from among blocks configuring the vegetation inspection apparatus 10 (FIG. 1) are depicted.

The sensor 103 measures (senses) an R channel value (D_Ref_env_R) and an IR channel value (D_Ref_env_IR) according to reflection light from the reference reflector plate 101 under a measurement light source and supplies the measured values to the correction processing unit 105. Further, the sensor 103 measures an R channel value (D_Plt_ISP_env_R) and an IR channel value (D_Plt_ISP_env_IR) according to reflection light from the inspection object 50 under the measurement light source and supplies the measured values to the correction processing unit 105.

The correction processing unit 105 refers to a lookup table (LUT) created in advance to determine a measurement light source correspondence correction gain according to a measurement spectral ratio (R/IR ratio) of the reference reflector plate 101 under the measurement light source, and corrects the measurement spectral ratio (R/IR ratio) of the inspection object 50 under the measurement light source using the determined measurement light source correspondence correction gain.

The correction processing unit 105 is configured from a correction gain processing unit 111, a storage unit 112 and a measurement light correction unit 113. It is to be noted that the storage unit 112 has stored therein in advance a lookup table (LUT) created by the lookup table creation process (FIG. 7).

The correction gain processing unit 111 performs a process relating to a correction gain of a spectral ratio (measurement light source correspondence correction gain). The correction gain processing unit 111 is configured from a reference reflector plate R/IR ratio calculation unit 121 and a correction gain determination unit 122.

The reference reflector plate R/IR ratio calculation unit 121 calculates a measurement spectral ratio (R_Ref_env) of the reference reflector plate 101 under the measurement light source using the R channel values (D_Plt_ISP_env_R) and the IR channel values (D_Plt_ISP_env_IR) supplied thereto from the sensor 103, and supplies the measurement spectral ratio (R_Ref_env) to the correction gain determination unit 122.

The correction gain determination unit 122 refers to the lookup table (LUT) stored in the storage unit 112 to determine a measurement light source correspondence correction gain (G_Plt_AVE_env) according to the measurement spectral ratio (R_Ref_env) (of the reference reflector plate 101 under the measurement light source) supplied from the reference reflector plate R/IR ratio calculation unit 121, and supplies the measurement light source correspondence correction gain (G_Plt_AVE_env) to the measurement light correction unit 113.

The measurement light correction unit 113 uses the correction gain of the spectral ratio (measurement light source correspondence correction gain) to perform a process for correcting the measurement spectral ratio (R/IR ratio) of the inspection object 50 under the measurement light source. The measurement light correction unit 113 is configured from an inspection object R/IR ratio calculation unit 123 and a gain correction unit 124.

The inspection object R/IR ratio calculation unit 123 calculates a measurement spectral ratio (R_Plt_ISP_env) of the inspection object 50 under the measurement light source using the R channel value (D_Plt_ISP_env_R) and the IR channel value (D_Plt_ISP_env_IR) supplied thereto from the sensor 103, and supplies the measurement spectral ratio (R_Plt_ISP_env) to the gain correction unit 124.

To the gain correction unit 124, a measurement light source correspondence correction gain (G_Plt_AVE_env) from (the correction gain determination unit 122 of) the correction gain processing unit 111 and a measurement spectral ratio (R_Plt_ISP_env) (of the inspection object 50 under the measurement light source) from the inspection object R/IR ratio calculation unit 123 are supplied. The gain correction unit 124 corrects the measurement spectral ratio (R_Plt_ISP_env) of the inspection object 50 under the measurement light source on the basis of the measurement light source correspondence correction gain (G_Plt_AVE_env). The measurement spectral ratio (R_Plt_ISP_env_comp) (of the inspection object 50 under the measurement light source) after corrected is supplied to the inspection index calculation unit 106.

The inspection index calculation unit 106 performs a process for calculating an inspection index (NDVI value) of the inspection object 50. The inspection index calculation unit 106 is configured from an NDVI value calculation unit 131.

The NDVI value calculation unit 131 calculates a normalized vegetation index (NDVI value) as an inspection index of the inspection object 50 on the basis of the measurement spectral ratio (R_Plt_ISP_env_comp) (of the inspection object 50 under the measurement light source) after corrected supplied thereto from (the gain correction unit 124 of the measurement light correction unit 113 of) the correction processing unit 105, and outputs the normalized vegetation index (NDVI value).

As described above, when an inspection index (NDVI value) of the inspection object 50 under the measurement light source is to be measured, the vegetation inspection apparatus 10 refers to a lookup table (LUT) to determine a measurement light source correspondence correction gain according to a measurement spectral ratio (R/IR ratio) of the reference reflector plate 101 under the measurement light source, corrects the measurement spectral ratio (R/IR ratio) of the inspection object 50 under the measurement light source in response to the determined measurement light source correspondence correction gain, and calculates an inspection index (NDVI value) from the measurement spectral ratio (R/IR ratio) (of the inspection object 50 under the measurement light source) after amended.

(First measurement process of inspection index)

Now, a first measurement process of an inspection index of the inspection object 50 under the measurement light source executed by the vegetation inspection apparatus 10 of FIG. 10 is described with reference to a flow chart of FIG. 11.

At step S111, the sensor 103 measures an R channel value (D_Ref_env_R) and an IR channel value (D_Ref_env_IR) of the reference reflector plate 101 under the measurement light source.

At step S112, the reference reflector plate R/IR ratio calculation unit 121 arithmetically operates an expression (40) given below on the basis of the R channel value (D_Ref_env_R) and the IR channel value (D_Ref_env_IR) measured by the process at step S111 to calculate a measurement spectral ratio (R_Ref_env) of the reference reflector plate 101 under the measurement light source.

[Math. 9]

$$R\_Ref\_env = D\_Ref\_env\_R/D\_Ref\_env\_IR \ ... \ (40)$$

At step S113, the correction gain determination unit 122 refers to the lookup table (LUT) stored in the storage unit 112 to search for an assumed light source of a color temperature at which the reference spectral ratio of the reference reflector plate 101 is closest to the measurement spectral ratio (R_Ref_env) (of the reference reflector plate 101 under the measurement light source) calculated by the process at step S112 from among the plurality of assumed light sources and determines the searched out assumed light source as the estimated light source.

At step S114, the correction gain determination unit 122 refers to the lookup table (LUT) stored in the storage unit 112 to determine a measurement light source correspondence correction gain (G_Plt_AVE_env) according to the estimated light source estimated by the process at step S113.

Here, the determination of a measurement light source correspondence correction gain includes a case in which a measurement light source correspondence correction gain is selected from among a plurality of correction gains stored in the lookup table (LUT) and another case in which a measurement light source correspondence correction gain is calculated by performing interpolation using values of correction gains stored in the lookup table (LUT).

For example, where the lookup table (LUT) described hereinabove with reference to FIG. 9 is stored in the storage unit 112, when the value of the measurement spectral ratio (R_Ref_env) (of the reference reflector plate 101 under the measurement light source) calculated by the process at step S112 is an equal value or the closest value to the reference spectral ratio (R_Ref_2800K) of the reference reflector plate 101 under an assumed light source of the color temperature of 2800K, the assumed light source of the color temperature of 2800K is determined as an estimated light source (S113). Then, from within the lookup table (LUT) of FIG. 9, a correction gain (G_Plt_AVE_2800K) according to the assumed light source of the color temperature of 2800K is selected as a measurement light source correspondence correction gain (S114).

Meanwhile, FIG. 12 depicts an example of particular numerical values of the lookup table (LUT). In the lookup table (LUT) of FIG. 12, with the assumed light source of the color temperature of 2800K, a reference spectral ratio (R_Ref_2800K) (of the reference reflector plate 101 under the assumed light source) of 0.25 and a correction gain (G_Plt_AVE_2800K) of 5.0 are associated. Similarly, with the assumed light source of the color temperature of 5500K, a reference spectral ratio (R_Ref_5500K) (of the reference reflector plate 101 under the assumed light source) of 1.0 and a correction gain (G_Plt_AVE_5500K) of 1.0 are associated, and with the assumed light source of the color temperature of 10000K, a reference spectral ratio (R_Ref_10000K) (of the reference reflector plate 101 under the assumed light source) of 4.0 and a correction gain (G_Plt_AVE_10000K) of 0.3 are associated.

Here, for example, where the value of the measurement spectral ratio (R_Ref_env) (of the reference reflector plate 101 under the measurement light source) calculated by the process at step S112 is 0.25, a value (0.25) coincident with the value calculated by the process at step S112 exists among values (0.25, 1.0 and 4.0) of the reference spectral ratio (of the reference reflector plate 101 under the assumed light source) corresponding to the assumed light sources of the color temperatures in the lookup table (LUT). Since the assumed light source at this time is the assumed light source of the color temperature of 2800K, this assumed light source is determined as the estimated light source. Then, 5.0 is selected as the correction gain (measurement light source correspondence correction gain) corresponding to the assumed light source of the color temperature of 2800K as the estimated light source.

It is to be noted that also a case is assumed in which a value coincident with the value of the measurement spectral ratio (R_Ref_env) (of the reference reflector plate 101 under the measurement light source) calculated by the process at step S112 does not exist among the values of the reference spectral ratio (of the reference reflector plate 101 under the assumed light source) of the lookup table (LUT). For example, where the value calculated by the process at step S112 (calculation value) is 0.4, a coincident value does not exist in the lookup table (LUT).

In this case, if any light source other than the assumed light sources upon creation of the lookup table (LUT) is to be excluded as an estimated light source, then the reason why a value coincident with the value calculated by the process at step S112 (calculation value) does not exist in the lookup table (LUT) is treated as a measurement error, and a value closest to the calculation value, namely, a value that indicates a minimum difference absolute value from among values in the lookup table (LUT) may be searched for. For example, since the closest value to the value (0.4) calculated by the process at step S112 from among the values (0.25, 1.0 and 4.0) of the reference spectral ratio (of the reference reflector plate 101 under the assumed light source) corresponding to the assumed light sources of the color temperatures is 0.25 in the lookup table (LUT), the assumed light source of the color temperature of 2800K is determined as an estimated light source. Then, 5.0 is selected as a correction gain (measurement light source correspondence correction gain) corresponding to the assumed light source of the color temperature of 2800K as the estimated light source.

On the other hand, where a light source other than the assumed light sources upon creation of the lookup table (LUT) is permitted as the estimated light source, also it is possible to treat the reason why a value coincident with the value calculated by the process at step S112 (calculation value) does not exist in the lookup table (LUT) as a fact that the measurement light source has an intermediate spectral characteristic between plural ones of the assumed light sources. For example, since the closest value and the second closest value in the lookup table (LUT) to the value (0.4) calculated by the process at step S112 are 0.25 and 1.0, respectively, by using difference absolute values between the values and the calculation value (0.4) as weights upon weighted average calculation, 4.2 can be calculated as an optimum correction gain (measurement light source correspondence correction gain) from the correction gains (5.0, 1.0) corresponding to the respective values.

$$5.0 \times ((1.0 - 0.4)/(1.0 - 0.25)) + 1.0 \times ((0.4 - 0.25)/(1.0 - 0.25)) = 4.2$$

It is to be noted that, although a flow of processing is described here that a light source estimation process is performed by the process at step S113 and a determination process of a measurement light source correspondence correction gain according to a result of the light source estimation is performed by the process at step S114, a measurement light source correspondence correction gain may otherwise be determined without performing the light source estimation process. In particular, a measurement spectral ratio (R_Ref_env) (of the reference reflector plate 101 under the measurement light source) calculated by the process at step S112 and reference spectral ratios (R_Ref_2800K, R_Ref_5500K, R_Ref_10000K) (of the reference reflector plate 101) stored in the lookup table (LUT) are directly compared with each other to determine a correction gain corresponding to the reference spectral ratio equal or closest to the measurement spectral ratio (R_Ref_env) as the measurement light source correspondence correction gain. In this case, since the spectral ratios are compared directly with each other, information relating to the assumed light sources may not necessarily be included in the lookup table (LUT).

Referring back to FIG. 11, at step S115, the sensor 103 measures an R channel value (D_Plt_ISP_env_R) and an IR channel value (D_Plt_ISP_env_IR) of the inspection object 50 under the measurement light source.

It is to be noted that, although a flow of processing is depicted in FIG. 11 that the vegetation inspection apparatus 10 measures the reference reflector plate 101 under the measurement light source by the process at step S111 and measures the inspection object 50 under the measurement light source by the process at step S115, when the reference reflector plate 101 and the inspection object 50 are to be measured at the same time, the process at step S115 is performed at a timing same as that of the process at step S111. In particular, the vegetation inspection apparatus 10 may perform the measurement processes at step S111 and step S115 at the same time or at different timings from each other. It is to be noted that the measurement processes at steps S111 and S115 are performed at timings before measurement results are required by the calculation processes at steps S112, S116 and so forth.

At step S116, the inspection object R/IR ratio calculation unit 123 arithmetically operates an expression (41) given below based on the R channel value (D_Plt_ISP_env_R) and the IR channel value (D_Plt_ISP_env_IR) measured by the process at step S115 to calculate a measurement spectral ratio (R_Plt_ISP_env) of the inspection object 50 under the measurement light source.
[Math. 10]

$$R\_Plt\_ISP\_env = D\_Plt\_ISP\_env\_R/D\_Plt\_ISP\_env\_IR$$

$$\dots \ (41)$$

At step S117, the gain correction unit 124 arithmetically operates an expression (42) given below using the measurement light source correspondence correction gain (G_Plt_AVE_env) determined by the process at steps S113 and S114 to correct the measurement spectral ratio (R_Plt_ISP_env) (of the inspection object 50 under the measurement light source) calculated by the process at step S116.
[Math. 11]

$$R\_Plt\_ISP\_env\_comp = R\_Plt\_ISP\_env \times G\_Plt\_AVE\_env$$

$$\dots \ (42)$$

In particular, it is assumed that, for example, where the assumed light source of the color temperature of 2800K is determined as the estimated light source and G_Plt_AVE_2800K is determined as the measurement light source correspondence correction gain regarding the estimated light source by the processes at steps S113 and S114, and, at step S116, an expression (43) given below is arithmetically operated to calculate R_Plt_ISP_2800K as the measurement spectral ratio (R_Plt_ISP_env) of the inspection object 50.
[Math. 12]

$$R\_Plt\_ISP\_2800K = D\_Plt\_ISP\_2800K\_R/D\_Plt\_ISP\_2800K\_IR$$

$$\dots \ (43)$$

In this case, if, at step S117, the measurement light source correspondence correction gain (G_Plt_AVE_2800K) determined by the processes at steps S113 and S114 is applied to R_Plt_ISP_2800K determined by the expression (43), then it can be represented by an expression (44) given below.
[Math. 13]

$$R\_Plt\_ISP\_2800K\_comp = R\_Plt\_ISP\_2800K \times G\_Plt\_AVE\_2800K$$
$$= R\_Plt\_ISP\_2800K \times$$
$$(R\_Plt\_AVE\_5500K/R\_Plt\_AVE\_2800K)$$

$$\dots \ (44)$$

Here, the inspection object 50 that is the measurement object at the present point of time is one of objects of a population of objects (inspection objects) whose spectral reflectance characteristic O_Plt_i($\lambda$) is measured upon calculation of the average spectral reflectance characteristic O_Plt_AVE($\lambda$) in creation of the lookup table (LUT). Therefore, it is considered that the spectral reflectance characteristic of the inspection object 50 of the measurement object and the average spectral reflectance characteristic O_Plt_AVE($\lambda$) in the spectral sensitivity band of the R channel and the IR channel have a high correlation therebetween. Therefore, the spectral ratio (R/IR ratio) of the inspection object 50 by a color temperature variation of the assumed light source (reference light source) has a strong correlation between that in the case of the spectral reflectance characteristic of the inspection object 50 of the measurement object and that in the case of the average spectral reflectance characteristic O_Plt_AVE($\lambda$). Accordingly, they have a relationship of an expression (45) given below.
[Math. 14]

$$R\_Plt\_AVE\_5500K/R\_Plt\_AVE\_2800K$$
$$\approx R\_Plt\_ISP\_5500K/R\_Plt\_ISP\_2800K \ ... \ (45)$$

Then, by applying the expression (45) to the expression (44), they have the relationship of an expression (46) given below.
[Math. 15]

$$R\_Plt\_ISP\_2800K\_comp \approx R\_Plt\_ISP\_2800K \times$$
$$(R\_Plt\_ISP\_5500K/R\_Plt\_ISP\_2800K)$$
$$= R\_Plt\_ISP\_5500K \ ... \ (46)$$

In particular, it is possible to correct the measurement spectral ratio (R_Plt_ISP_2800K) of the inspection object 50 under the measurement light source of the color temperature of 2800K to the measurement spectral ratio (R_Plt_ISP_5500K) of the inspection object 50 under the light source (reference light source) of the reference color temperature of 5500K. It is to be noted that, while the measurement light source of the color temperature of 2800K is described here, also under a measurement light source of any other color temperature, if similar measurement, correction and so forth are performed, then a measurement spectral ratio of the inspection object 50 under a measurement light source can always be corrected to a measurement spectral ratio of the inspection object 50 under a light source of a reference color temperature (reference light source).

At step S118, the NDVI value calculation unit 131 arithmetically operates an expression (47) given below using the measurement spectral ratio (R_Plt_ISP_nv_comp) (of the inspection object 50 under the measurement light source) corrected by the process at step S117 to calculate a normalized vegetation index (NDVI value) as the inspection index of the inspection object 50.
[Math. 16]

$$NDVI = (1 - R\_Plt\_ISP\_env\_comp)/(1 + R\_Plt\_ISP\_env\_comp) \ ... \ (47)$$

When the process at step S118 ends, the first measurement process of an inspection index of FIG. 11 is ended.

The first measurement process of an inspection index has been described. In this first measurement process of an inspection index, by referring to a lookup table (LUT), a measurement light source correspondence correction gain (G_Plt_AVE_env) according to a measurement spectral ratio (R_Plt_AVE_env) of the reference reflector plate 101 under the measurement light source is determined. Then, a measurement spectral ratio (R_Plt_ISP_env) of the inspection object 50 under the measurement light source is corrected using the measurement light source correspondence correction gain (G_Plt_AVE_env), and an inspection index (NDVI value) of the inspection object 50 is calculated using the measurement spectral ratio (R_Plt_ISP_env_comp) after correction.

Here, if the correction process (measurement light correction) at step S117 described above is not performed but the measurement spectral ratio (R_Plt_ISP_2800K) of the inspection object 50 under the measurement light source of the color temperature of 2800K is directly used to calculate a normalized vegetation index (NDVI value) of the inspection object 50, then the NDVI value is represented by an expression (48).
[Math. 17]

$$NDVI\_Plt\_ISP\_2800K$$
$$= (1 - R\_Plt\_ISP\_2800K)/(1 + R\_Plt\_ISP\_2800K)$$
$$... \ (48)$$

Here, as apparent from the relationship of the expression (48), if this expression (48) is arithmetically operated to determine a normalized vegetation index (NDVI value), then the normalized vegetation index (NDVI value) varies in response to a variation of the color temperature of the measurement light source. On the other hand, if the correction process (measurement light correction) at step S117 described hereinabove is performed to calculate a normalized vegetation index (NDVI value) of the inspection object 50 using the measurement spectral ratio (R_Plt_ISP_2800K_comp) (of the inspection object 50 under the measurement light source) after corrected, then the NDVI value is represented by an expression (49).
[Math. 18]

$$\begin{aligned}
\mathrm{NDVI\_Plt\_ISP\_2800K\_comp} \\
&= (1 - \mathrm{R\_Plt\_ISP\_2800K\_comp})/(1 + \mathrm{R\_Plt\_ISP\_2800K\_comp}) \\
&\approx (1 - \mathrm{R\_Plt\_ISP\_5500K})/(1 + \mathrm{R\_Plt\_ISP\_5500K}) \\
&= \mathrm{NDVI\_Plt\_ISP\_5500K} \quad ... \quad (49)
\end{aligned}$$

In particular, as apparent from the relationship of the expression (49), if the expression (48) is arithmetically operated to determine a normalized vegetation index (NDVI value), then the normalized vegetation index (NDVI value) is always determined in accordance with the measurement spectral ratio (R_Plt_ISP_5500K) of the inspection object 50 under the light source (reference light source) of the reference color temperature of 5500K irrespective of a variation of the color temperature of the measurement light source. Consequently, since it becomes possible for the normalized vegetation index (NDVI value) to have a value equal to or approximated with a high degree of accuracy to a normalized vegetation index (NDVI value) obtained from the measurement spectral ratio (R_Plt_ISP_5500K) of the inspection object 50 under the light source (reference light source) of the reference color temperature of 5500K, measurement of a normalized vegetation index (NDVI value) of a high degree of accuracy, which does not rely upon a variation of the color temperature of the measurement light source, becomes possible. Since the light source dependency can be removed (excluded) well in this manner, accurate measurement light correction can be performed. For example, also where the light source varies depending upon a variation in weather such as a fine weather, a cloudy weather or a rainy weather, in regard to a same inspection object 50 (for example, a plant such as a turf), an inspection index (NDVI value) of the same value is measured.

(Second functional configuration of vegetation inspection apparatus)

FIG. 13 is a view depicting a second functional configuration of the vegetation inspection apparatus 10 (FIG. 1) upon measurement of an inspection index (NDVI value) of the inspection object 50 under a measurement light source.

The vegetation inspection apparatus 10 of FIG. 13 is different, in comparison with the configuration of the vegetation inspection apparatus 10 described hereinabove with reference to FIG. 10, in configuration of the inspection index calculation unit 106. In particular, the inspection index calculation unit 106 of FIG. 13 additionally includes, at a preceding stage to the NDVI value calculation unit 131, a normalization unit 132 and a storage unit 133 for performing normalization of light source-sensor components for a measurement spectral ratio (R_Plt_ISP_env_comp) (of the inspection object 50 under the measurement light source) after corrected.

Here, as regards the reason why such normalization is performed, a vegetation index such as a normalized vegetation index (NDVI value) is generally defined only by a spectral reflectance characteristic of an object without relying upon the measurement system of a light source and a sensor. In other words, the vegetation index is defined only by a characteristic of an object from between characteristics of a light source (reference light source), a sensor (sensor 103) and an object (inspection object 50).

Accordingly, it is desirable to exclude a spectral characteristic of the reference light source (for example, a factor that a spectrum of a reference light source of the reference color temperature of 5500K is displaced from a flat spectrum) and a spectral characteristic of the sensor 103 (for example, a sensitivity ratio between the R channel and the IR channel). Therefore, a spectral ratio (R_Flat_5500K) for normalization with only the spectral characteristic of the reference light source and the spectral sensitivity characteristic of the sensor 103 taken into consideration is calculated (measured) in advance and stored into the storage unit 133. Consequently, by dividing the measurement spectral ratio (R_Plt_ISP_env_comp) (of the inspection object 50 under the measurement light source) after corrected by the spectral ratio (R_Flat_5500K) for normalization, the components of the light source (reference light source) and the sensor (sensor 103) are normalized.

However, if the R channel value and the IR channel value where measurement (sensing) of an object by a flat spectrum under the reference light source is assumed are represented by D_Flat_5500K_R and D_Flat_5500K_IR, respectively, then the spectral ratio (R_Flat_5500K) for normalization can be determined by arithmetically operating an expression (50) given below.
[Math. 19]

$$\mathrm{R\_Flat\_5500K} = \mathrm{D\_Flat\_5500K\_R}/\mathrm{D\_Flat\_5500K\_IR} \quad ... \quad (50)$$

Referring back to FIG. 13, the normalization unit 132 reads out information indicative of the spectral ratio (R_Flat_5500K) for normalization stored in the storage unit 133. The normalization unit 132 normalizes the measurement spectral ratio (R_Plt_ISP_env_comp) (of the inspection object 50 under the measurement light source) after corrected supplied thereto from (the gain correction unit 124 of the measurement light correction unit 114 of) the correction processing unit 105 on the basis of the spectral ratio (R_Flat_5500K) for normalization read out from the storage unit 133, and supplies the measurement spectral ratio (R_Plt_ISP_norm) (of the inspection object 50 under the measurement light source) after normalized to the NDVI value calculation unit 131.

The NDVI value calculation unit 131 calculates a normalized vegetation index (NDVI value) as the inspection index of the inspection object 50 on the basis of the measurement spectral ratio (R_Plt_ISP_norm) (of the inspection object 50 under the measurement light source) after normalized supplied from the normalization unit 132, and outputs the normalized vegetation index (NDVI value).

It is to be noted that, although the storage unit 112 of the correction processing unit 105 and the storage unit 133 of the inspection index calculation unit 106 in FIG. 13 are configured as storage units separate from each other for the convenience of description, they may otherwise be configured as a single storage unit.

(Second measurement process of inspection index)

Now, a second measurement process of an inspection index of the inspection object 50 under the measurement light source, which is executed by the vegetation inspection apparatus 10 of FIG. 13, is described with reference to a flow chart of FIG. 14.

At steps S131 to S137, a measurement light source correspondence correction gain (G_Plt_AVE_env) according to the measurement spectral ratio (R_Ref_env) of the reference reflector plate 101 under the measurement light source is determined, and a measurement spectral ratio (R_Plt_ISP_env) of the inspection object 50 under the measurement light source is corrected using the measurement light source correspondence correction gain (G_Plt_AVE_env) similarly as at steps S111 to S117 of FIG. 11.

It is to be noted that, while also FIG. 14 indicates a flow of such processing that the vegetation inspection apparatus 10 measures the reference reflector plate 101 under the measurement light source in the process at step S131 and then measures the inspection object 50 under the measurement light source in the process at step S135, where the reference reflector plate 101 and the inspection object 50 are measured at the same time, the process at step S135 is performed at a timing same as that of the process at step S131. In particular, the vegetation inspection apparatus 10 may perform the measurement processes at step S131 and step S135 simultaneously or at different timings. It is to be noted that the measurement processes at step S131 and step S135 are performed at timings before the measurement results are required by the calculation processes at steps S132, S136 and so forth.

At step S138, the normalization unit 132 performs normalization of light source-sensor components in regard to the measurement spectral ratio (R_Plt_ISP_env_comp) (of the inspection object 50 under the measurement light source) corrected by the process at step S137.

In particular, the normalization unit 132 reads out information indicative of the spectral ratio (R_Flat_5500K) for normalization stored in the storage unit 133 in advance. Then, the normalization unit 132 arithmetically operates an expression (51) given below using the spectral ratio (R_Flat_5500K) for normalization read out from the storage unit 133 to perform normalization of the light source-sensor components with regard to the measurement spectral ratio (R_ISP_env_comp) (of the inspection object 50 under the measurement light source) after corrected.

[Math. 20]

$$R\_Plt\_ISP\_norm = R\_Plt\_ISP\_env\_comp/R\_Flat\_5500K$$

$$\qquad\qquad\qquad\qquad ... \quad (51)$$

At step S139, the NDVI value calculation unit 131 arithmetically operates an expression (52) given below using the measurement spectral ratio (R_Plt_ISP_norm) (of the inspection object 50 under the measurement light source) normalized by the process at step S138 to calculate a normalized vegetation index (NDVI value) as an inspection index of the inspection object 50.

[Math. 21]

$$NDVI\_norm = (1 - R\_Plt\_ISP\_norm)/(1 + R\_Plt\_ISP\_norm)$$

$$\qquad\qquad\qquad\qquad ... \quad (52)$$

The second measurement process of an inspection index has been described. In this second measurement process of an inspection index, a measurement light source correspondence correction gain (G_Plt_AVE_env) according to the measurement spectral ratio (R_Ref_env) of the reference reflector plate 101 under the measurement light source, and the measurement spectral ratio (R_Plt_ISP_env) of the inspection object 50 under the measurement light source is corrected using the measurement light source correspondence correction gain (G_Plt_AVE_env). Then, the measurement spectral ratio (R_Plt_ISP_env_comp) after corrected is normalized, and the inspection index (NDVI value) of the inspection object 50 is calculated using the measurement spectral ratio (R_Plt_ISP_norm) after normalized.

<5. Modifications>

(Example of attachment of reference reflector plate)

Although the reference reflector plate 101 can be installed at a position at which it can be measured by the sensor 103, for example, it can be attached to a predetermined position in front of a camera in which the sensor 103 is incorporated. FIG. 15 is a view depicting an example in a case in which the reference reflector plate 101 is attached in front of a camera.

In A of FIG. 15, a bar-like member is attached to a camera as the vegetation inspection apparatus 10, and a reference reflector plate 101 having a circular shape is attached to a distal end of the bar-like member (end at the object side). Meanwhile, B of FIG. 15 depicts that, as an object that is to be measured (sensed) by the camera as the vegetation inspection apparatus 10, a turf (lawn) of a stadium that is an inspection object 50 and the reference reflector plate 101 having a circular shape exist within the same angle of view.

In such a state that the inspection object 50 and the reference reflector plate 101 can be measured at the same time, a measurement process (FIGS. 11 and 14) of an inspection index of the inspection object 50 under the measurement light source L1 is executed. Consequently, only by measuring (sensing) the turf (lawn) of the stadium and the reference reflector plate 101 at the same time, for example, by the camera as the vegetation inspection apparatus 10, a normalized vegetation index (NDVI value) of the turf in the angle of view of the camera is obtained. Therefore, by displaying a result of this, the state of the turf of the stadium can be confirmed rapidly.

It is to be noted that, while it is described that, in FIG. 15, the shape of the reference reflector plate 101 is a circular shape, the shape of the reference reflector plate 101 may be a different shape such as, for example, an elliptical shape or a quadrangular shape.

Further, as the camera (vegetation inspection apparatus 10) described above, for example, a multispectral camera can be used by which a multispectral signal is obtained by dispersing light (reflection light) incident through a lens into a plurality of wavelength bands and forming an image of the light in the wavelength bands on a two-dimensional sensor face. Further, for the camera (vegetation inspection apparatus 10) described above, a camera having a spectral sensing function such as, for example, a so-called hyperspectral camera can be used in addition to the multispectral camera.

(Inspection object and inspection index)

While, in the foregoing description, the inspection object 50 is described taking a plant (for example, a turf or the like) as an example, the inspection object 50 may be an object other than a plant. For example, by measuring, in a manufacturing factory of food, an inspection index of food manufactured by the inspection apparatus (vegetation inspection apparatus 10) of the present technology as the inspection object 50, an inspection index of food can be measured without relying upon a light source in the factory. Consequently, even if the illumination light in the factory varies, ranking of food can be performed.

Further, while, in the foregoing description, the inspection index when a plant is set as the inspection object 50 is described taking a normalized vegetation index (NDVI value) as an example, a vegetation index different from the normalized vegetation index (NDVI value) may be measured. For example, as the different vegetation index, a ratio vegetation index (RVI: Ratio Vegetation Index), a difference vegetation index (DVI: Difference Vegetation Index) and so forth can be used.

Here, the ratio vegetation index (RVI value) is calculated by arithmetically operating an expression (53) given below.

$$RVI = IR/R \quad ... \quad (53)$$

Meanwhile, the difference vegetation index (DVI value) is calculated by arithmetically operating an expression (54) given below.

$$DVI = IR - R \ ... \ (54)$$

It is to be noted that, in the expression (53) and the expression (54), IR represents a reflectance in the near infrared region, and R represents a reflectance in the visible region (red). It is to be noted that, although only vegetation indexes in which IR and R are used as parameters are exemplified here, it is naturally possible to measure a different vegetation index using a reflectance of light in a visible region other than red or the like as a parameter. The spectral ratio is not limited to the combination of R and IR. From the sensor 103, components of other wavelength bands such as G or B other than R and IR may be outputted as outputs of RGBIR.

(Other embodiments)

It is to be that, although the foregoing description describes a case in which a lookup table (LUT) for one kind of inspection objects 50 (individuals having different characteristics) is prepare in advance and, upon measurement of an inspection index of an inspection object 50, the lookup table (LUT) is referred to, a lookup table (LUT) for each of a plurality of kinds of inspection objects 50 may be created by the lookup table creation apparatus 20. Then, in the vegetation inspection apparatus 10, if the lookup tables (LUTs) for the plurality of kinds of inspection objects 50 are stored into the storage unit 112, then since it becomes possible for the correction gain determination unit 122 to switch the lookup table (LUT) to be referred to in response to the kind of the inspection object 50, an appropriate correction gain (measurement light source correspondence correction gain) can be determined for any kind of inspection object 50.

While the foregoing description exemplifies a case in which assumed light sources of two or three color temperatures are used as the assumed light sources, various assumed light sources can be used such that assumed light sources of four or more color temperatures (other color temperatures) are used or light sources having different spectral characteristics are distinguished from each other although they have an equal color temperature, for example, like daylight and artificial light.

While the foregoing description exemplifies an average spectral characteristic (average spectral reflectance characteristic O_PLt_AVE($\lambda$)) as a representative spectral characteristic of an inspection object upon creation of a lookup table (LUT), a spectral characteristic different from an average spectral characteristic may be used. Further, although a case is exemplified in which, upon creation of a lookup table (LUT), measurement of a spectral characteristic (spectral reflectance characteristic O_Ref($\lambda$)) of the reference reflector plate is performed so as to establish a known state of the spectral characteristic, it is possible, for example, to use a white or gray reflector plate as the reference reflector plate such that, even if measurement of the spectral characteristic of the same is not performed, similar measurement light correction can be performed assuming that they have no wavelength dependency of the spectral characteristic.

While the foregoing description exemplifies measurement in which two sensor output values of the R channel and the IR channel are used, it is possible to use sensor output values having three or more spectral sensitivity bands different from each other or to use sensor output values only of visible rays. For example, where a measurement value corresponding to an optical filter that passes red light R, a measurement value corresponding to an optical filter that passes first infrared light IR1 and a measurement value corresponding to an optical filter that passes second infrared light RI2 are utilized in the sensor 103 in FIG. 1, for example, two spectral ratios of IR1/R and IR2/R are used to create lookup tables (LUTs) or, upon measurement of an inspection index of the inspection object 50, measurement of the inspection object 50 and the reference reflector plate 101 or light source estimation is performed.

(Different configuration example of vegetation inspection apparatus)

FIG. 16 depicts a configuration in which the vegetation inspection apparatus 10 includes a sensor 103, a correction processing unit 105 and an inspection index calculation unit 106. This configuration corresponds to the configuration of FIG. 10 described hereinabove and is a configuration in the case in which the vegetation inspection apparatus 10 has all functions of the sensor 103, correction processing unit 105, inspection index calculation unit 106 and so forth.

While, in the foregoing description, a case is described in which the vegetation inspection apparatus 10 has all functions like the configuration depicted in FIG. 16, part of the functions of the vegetation inspection apparatus 10 (FIG. 1) may be had by some other apparatus. In the following, a configuration in which a different apparatus has part of the functions of the vegetation inspection apparatus 10 (FIG. 1) is describe with reference to FIGS. 17 to 19. It is to be noted that, in FIGS. 17 to 19, like portions to the blocks configuring FIG. 10 described hereinabove are denoted by like reference characters, and description of them is suitably omitted. Further, this similarly applies also the configuration of FIG. 13 though not depicted.

(First configuration example of vegetation inspection system)

FIG. 17 is a view depicting a first example of a configuration of the vegetation inspection system.

Referring to FIG. 17, the vegetation inspection system 11 is configured from a measurement apparatus 60 and a processing apparatus 70. Here, both the measurement apparatus 60 and the processing apparatus 70 have a communication function and can exchange data utilizing wireless communication or wired communication in compliance with a predetermined standard. For example, where the processing apparatus 70 is a server provided on the Internet, the measurement apparatus 60 accesses the processing apparatus 70 through the Internet and transmits data to the processing apparatus 70.

The measurement apparatus 60 is configured from a sensor 103, a correction processing unit 105 and a communication unit 107. Further, the correction processing unit 105 is configured from a correction gain processing unit 111, a storage unit 112, and a measurement light correction unit 113.

The correction gain processing unit 111 refers to a lookup table (LUT) stored in the storage unit 112 to determine a correction gain (measurement light source correspondence correction gain) according a measurement spectral ratio (R/IR ratio) of the reference reflector plate 101 calculated from an output value of the sensor 103 and supplies the correction gain to the measurement light correction unit 113.

The measurement light correction unit 113 uses the correction gain (measurement light source correspondence correction gain) from the correction gain processing unit 111 to correct the measurement spectral ratio (R/IR ratio) of the inspection object 50 calculated from an output value of the sensor 103 and supplies the resulting spectral ratio (R/IR ratio) to the communication unit 107. The communication unit 107 transmits information indicative of the measurement spectral ratio (R/IR ratio) (of the inspection object 50) after corrected supplied from the measurement light correction unit 113 to the processing apparatus 70 utilizing, for example, wireless communication or the like.

The processing apparatus 70 is configured from an inspection index calculation unit 106 and a communication unit 108. Further, the inspection index calculation unit 106 is configured from an NDVI value calculation unit 131. The communication unit 108 receives information transmitted thereto from the measurement apparatus 60 and indicative of the measurement spectral ratio (R/IR ratio) (of the inspection object 50) after corrected and supplies the received information to the NDVI value calculation unit 131. The NDVI value calculation unit 131 uses the information supplied from the communication unit 108 and indicative of the measurement spectral ratio (R/IR ratio) (of the inspection object 50) after corrected to calculate a normalized vegetation index (NDVI value) as an inspection index of the inspection object 50 and outputs the normalized vegetation index (NDVI value).

(Second configuration example of vegetation inspection system)

FIG. 18 is a view depicting a second configuration example of the vegetation inspection system.

Referring to FIG. 18, the vegetation inspection system 11 is configured from a measurement processing apparatus 80 and a storage apparatus 90. Here, both the measurement processing apparatus 80 and the storage apparatus 90 have a communication function and can exchange data utilizing wireless communication or wired communication in compliance with a predetermined standard. For example, where the storage apparatus 90 is a server provided on the Internet, the measurement processing apparatus 80 accesses the storage apparatus 90 through the Internet and receives data from the storage apparatus 90.

The measurement processing apparatus 80 is configured from a sensor 103, a correction processing unit 105, an inspection index calculation unit 106 and a communication unit 107. Further, the correction processing unit 105 is configured from a correction gain processing unit 111 and a measurement light correction unit 113. The inspection index calculation unit 106 is configured from an NDVI value calculation unit 131.

The communication unit 107 receives, upon measurement of the inspection object 50, a lookup table (LUT) from the storage apparatus 90, for example, utilizing wireless communication or the like and supplies the lookup table (LUT) to the correction gain processing unit 111. The correction gain processing unit 111 refers to the lookup table (LUT) supplied thereto from the communication unit 107 to determine a correction gain (measurement light source correspondence correction gain) according to a measurement spectral ratio (R/IR ratio) of the reference reflector plate 101 calculated from an output value of the sensor 103 and supplies the correction gain to the measurement light correction unit 113.

The measurement light correction unit 113 corrects the measurement spectral ratio (R/IR ratio) of the inspection object 50 calculated from an output value of the sensor 103 using the correction gain (measurement light source correspondence correction gain) from the correction gain processing unit 111 and supplies the corrected measurement spectral ratio (R/IR ratio) to the inspection index calculation unit 106. The NDVI value calculation unit 131 uses information supplied from the measurement light correction unit 113 and indicative of the measurement spectral ratio (R/IR ratio) (of the inspection object 50) after corrected to calculate a normalized vegetation index (NDVI value) as an inspection index of the inspection object 50 and outputs the normalized vegetation index (NDVI value).

For example, where a lookup table (LUT) is created by the lookup table creation apparatus 20, the lookup table (LUT) created by the lookup table creation apparatus 20 is stored into the storage apparatus 90. However, the lookup table creation apparatus 20 may otherwise provide the lookup table (LUT) directly to the measurement processing apparatus 80. Or, the storage apparatus 90 may store a spectral ratio for normalization in addition to the lookup table (LUT). Also it is possible to divert the lookup table (LUT) or the spectral ratio for normalization to a separate apparatus different from the measurement processing apparatus 80.

(Third configuration example of vegetation inspection system)

FIG. 19 is a view depicting a third configuration example of the vegetation inspection system.

Referring to FIG. 19, the vegetation inspection system 11 is configured from a measurement apparatus 60 and processing apparatus 70. Here, both the measurement apparatus 60 and the processing apparatus 70 have a communication function and can exchange data utilizing wireless communication or wired communication in compliance with a predetermined standard. For example, where the processing apparatus 70 is a server provided on the Internet, the measurement apparatus 60 accesses the processing apparatus 70 through the internet and transmits data to the processing apparatus 70.

The measurement apparatus 60 is configured from a sensor 103 and a communication unit 107. The sensor 103 measures the R channel value and the IR channel value of the reference reflector plate 101 and the inspection object 50 and supplies the measured values to the communication unit 107. The communication unit 107 transmits the R channel values and the IR channel values supplied from the sensor 103 to the processing apparatus 70, for example, utilizing wireless communication or the like.

The processing apparatus 70 is configured from a correction processing unit 105, an inspection index calculation unit 106 and a communication unit 108. Further, the correction processing unit 105 is configured from a correction gain processing unit 111, a storage unit 112 and a measurement light correction unit 113. The inspection index calculation unit 106 is configured from an NDVI value calculation unit 131.

The communication unit 108 receives R channel values and IR channel values of the reference reflector plate 101 and the inspection object 50 transmitted from the measurement apparatus 60, and supplies the R channel value and the IR channel value of the reference reflector plate 101 to the correction gain processing unit 111 and supplies the R channel value and the IR channel value of the inspection object 50 to the measurement light correction unit 113.

The correction gain processing unit 111 refers to a lookup table (LUT) stored in the storage unit 112 to determine a correction gain (measurement light source correspondence correction gain) according to a measurement spectral ratio (R/IR ratio) of the reference reflector plate 101 calculated from the channel values from the communication unit 108 and supplies the correction gain to the measurement light correction unit 113.

The measurement light correction unit 113 uses the correction gain (measurement light source correspondence correction gain) from the correction gain processing unit 111 to correct a measurement spectral ratio (R/IR ratio) of the inspection object 50 calculated from the channel values from the communication unit 108 and supplies the corrected measurement spectral ratio (R/IR ratio) to the NDVI value calculation unit 131. The NDVI value calculation unit 131 uses information supplied from the communication unit 108 and indicative of the measurement spectral ratio (R/IR ratio) (of the inspection object 50) after corrected to calculate a normalized vegetation index (NDVI value) as an inspection index of the inspection object 50 and outputs the normalized vegetation index (NDVI value).

It is to be noted that the term system signifies a logical set of a plurality of apparatus. Further, the configurations of FIGS. 17 to 19 are an example of configurations in the case where a different apparatus has part of the functions the vegetation inspection apparatus 10 has, and also it is possible to adopt a different configuration. For example, in the configurations of FIGS. 18 and 19, it is possible for (the NDVI value calculation unit 131 of) the inspection index calculation unit 106 to be provided by a different apparatus.

Further, in the vegetation inspection apparatus 10 of FIG. 16 or (the processing apparatus 70 or the measurement processing apparatus 80 of) the vegetation inspection systems 11 of FIGS. 17 to 19, a display controlling unit (not detected) may be provided at a succeeding stage to the inspection index calculation unit 106 such that the display controlling unit controls a display unit (not depicted) to display an NDVI image on the basis of a normalized vegetation index (NDVI value) calculated by (the NDVI value calculation unit 131 of) the inspection index calculation unit 106. However, the display unit that displays an NDVI may be provided in the inside of (the processing apparatus 70 or the measurement processing apparatus 80 of) the vegetation inspection apparatus 10 or the vegetation inspection system 11 or may be a display apparatus (not depicted) provided outside the apparatus mentioned.

(Particular example of measurement apparatus)

FIG. 20 exemplifies, as particular examples of the measurement apparatus 60 of FIG. 17 or 19, a fixed point measurement apparatus 60A that performs fixed point observation, a moving measurement apparatus 60B that performs moving observation and a satellite measurement apparatus 60C that performs measurement from an artificial satellite.

The fixed point measurement apparatus 60A depicted in A of FIG. 20 is fixed at a position, at which the inspection object 50 can be measured (sensed), by fixed legs 61A, and transmits a measurement signal (measurement value) measured there to the processing apparatus 70 (FIG. 17 or 19), for example, utilizing wireless communication or the like. The processing apparatus 70 can determine an inspection index (NDVI value) of the inspection object 50 measured by fixed point measurement by the fixed point measurement apparatus 60A by processing the measurement signal transmitted thereto from the fixed point measurement apparatus 60A.

The moving measurement apparatus 60B depicted in B of FIG. 20 is, for example, an unmanned aerial vehicle (UAV: Unmanned Aerial Vehicle), and flies by rotation of a rotary wing 61B in the form of a propeller and measures (shooting from a high level) the inspection object 50 from the sky. The moving measurement apparatus 60B transmits a measurement signal (measurement value) measured there utilizing, for example, wireless communication or the like to the processing apparatus 70 (FIG. 17 or 19). The processing apparatus 70 can determine an inspection index (NDVI value) of the inspection object 50 measured by moving measurement by the moving measurement apparatus 60B by processing the measurement signal transmitted thereto from the moving measurement apparatus 60B.

It is to be noted that the moving measurement apparatus 60B may store, for example, a flight route in advance as coordinate data such that it autonomously flies using position information of the GPS (Global Positioning System) or the like in addition to radio control. Further, while it is described that the moving measurement apparatus 60B in B of FIG. 20 is a rotary wing aircraft having the rotary wing 61B, the moving measurement apparatus 60B may be a fixed wind aircraft.

The satellite measurement apparatus 60C depicted in C of FIG. 20 is installed in an artificial satellite 61C. In the artificial satellite 61C, a measurement signal obtained by measurement (image pickup from the artificial satellite 61C) by the satellite measurement apparatus 60C (for example, a measurement value according to a satellite image) is transmitted to the processing apparatus 70 (FIG. 17 or 19) through a predetermined communication route. The processing apparatus 70 can determine an inspection index (NDVI value) of the inspection object 50 measured from the artificial satellite 61C by processing a measurement signal transmitted thereto from the satellite measurement apparatus 60C.

(Measurement using reference transmission plate)

Incidentally, while the description given above describes a case in which an inspection index (NDVI value) of the inspection object 50 is calculated by the vegetation inspection apparatus 10 utilizing the reference reflector plate 101, it is possible to utilize a transmittance characteristic in place of a reflectance characteristic of the reference reflector plate 101.

For example, by using a transmission filter having a spectral transmittance characteristic close to a spectral reflectance characteristic of the inspection object 50 (hereinafter referred to as reference transmission plate), it is possible to determine a correction gain (measurement light source correspondence correction gain), correct the measurement spectral ratio (R/IR ratio) of the inspection object 50 under the measurement light source and remove the light source dependency upon measurement of an inspection index (NDVI value) of the inspection object 50 similarly as in the case where the reference reflector plate 101 is used.

While the foregoing description describes that a lookup table (LUT) in which an assumed spectral ratio (R/IR ratio) of the reference reflector plate 101 and a correction gain are associated with each other for each of assumed light sources of different color temperatures is created in advance by the lookup table creation process (FIGS. 6 to 9), a lookup table (LUT) in which an assumed spectral ratio (R/IR ratio) of a reference transmission plate and a correction gain are associated with each other can be created similarly as in the lookup table creation process.

In particular, in the lookup table creation process (FIGS. 6 to 9), an R channel value and an IR channel value when reflection light of the reference reflector plate 101 is measured are calculated (S16), and an assumed spectral ratio (R/IR ratio) of the reference reflector plate 101 under the assumed light source of each color temperature is calculated (S17). Here, if a reference transmission plate is used, then an R channel value and an IR channel value when transmission light of the reference transmission plate is measured may be calculated in a similar manner as in the case in which the reference reflector plate 101 is used to calculate an assumed spectral ratio (R/IR ratio) of the reference transmission plate under the assumed light source of each color temperature.

Further, in the lookup table creation process (FIGS. 6 to 9), a correction gain according to an assumed spectral ratio (R/IR ratio) of the inspection object 50 is calculated for each assumed light source (S18 to S20) and the assumed spectral ratio (R/IR ratio) of the reference reflector plate 101 and the correction gain are associated with each other to create a lookup table (LUT) (S21). Here, where a reference transmission plate is used, a correction gain according to an assumed spectral ratio (R/IR ratio) of the inspection object 50 may be calculated for each assumed light source and the assumed spectral ratio (R/IR ratio) of the reference transmission plate and the correction gain may be associated with each other to create a lookup table (LUT) similarly as in the case where the reference reflector plate 101 is used.

In the following, a configuration in the case where, when a reference transmission plate is used in place of the reference reflector plate 101, the lookup table (LUT) corresponding to the reference transmission plate created by the lookup table creation process described above is used to perform measurement of an inspection index (NDVI value) of the inspection object 50 is described.

However, since it is necessary, in measurement in which a reference transmission plate is used, to provide a sensor 103 for measuring transmission light from the reference transmission plate, a plurality of sensors 103 are required. In this case, a configuration in which the plurality of sensors 103 are accommodated in the same housing (hereinafter referred to as monocular configuration) and another configuration in which the plurality of sensors 103 are accommodated in different housings from each other (hereinafter referred to as compound eye configuration). In the following description, the monocular configuration and the compound eye configuration are described in order.

(1) Monocular Configuration

(Configuration of vegetation inspection apparatus)

FIG. 21 is a perspective view depicting an example of a configuration of an appearance of a vegetation inspection apparatus where the monocular configuration is adopted.

Referring to FIG. 21, in the vegetation inspection apparatus 12, a light incidence section 12A and another light incidence section 12B are formed at a left side face side and an upper face side of a housing thereof such that light is introduced therethrough. Further, a sensor 103-1 and another sensor 103-2 are provided in the inside of the vegetation inspection apparatus 12. It is to be noted that, although the light incidence section 12A and the light incidence section 12B are each formed in a cylindrical shape that is hollow in the inside thereof, they have a role also as a cover.

In particular, in the vegetation inspection apparatus 12, light from the light incidence section 12A side (reflection light) enters along an optical axis La and is received by a sensor face of the sensor 103-1. Then, the sensor 103-1 measures an R channel value and an IR channel value according to the light (reflection light) from the inspection object 50 and outputs a measurement signal (measurement values) obtained as a result of the measurement to the correction processing unit 105 at a succeeding stage.

Further, in the vegetation inspection apparatus 12, light (sun light) from the light incidence section 12B side enters along an optical axis Lb.

Here, in the inside of a circle C represented by a broken line in FIG. 21, part of the vegetation inspection apparatus 12 at the light incidence section 12B side is depicted in an enlarged scale. As depicted here, a reference transmission plate 151 is provided on the optical axis Lb at the inner side of the light incidence section 12B having a cylindrical shape. It is to be noted that, in the light incidence section 12B, the reference transmission plate 151 is attached in a direction of an arrow mark in the figure.

In particular, light (sun light) from the light incidence section 12B side is transmitted by the reference transmission plate 151, and the transmission light is received by the sensor face of the sensor 103-2. Then, the sensor 103-2 measures an R channel value and an IR channel value according to the light transmitted by the reference transmission plate 151 (transmission light) and outputs a measurement signal (measurement values) obtained as a result of the measurement to the correction processing unit 105 at the succeeding stage.

Since the vegetation inspection apparatus 12 adopts the monocular configuration as described above, reflection light reflected by the inspection object 50 and transmission light transmitted by the reference transmission plate 151 are measured by the sensor 103-1 and the sensor 103-2 provided in the same housing, respectively. Now, a detailed internal configuration of the vegetation inspection apparatus 12 depicted in FIG. 21 is described with reference to FIG. 22.

Referring to FIG. 22, the vegetation inspection apparatus 12 is configured from a reference transmission plate 151, a lens 102, a sensor 103-1 and another sensor 103-2, an exposure controlling unit 104-1 and another exposure controlling unit 104-2, a correction processing unit 105 and an inspection index calculation unit 106.

It is to be noted that, in the vegetation inspection apparatus 12 of FIG. 22, portions same as the blocks configuring the vegetation inspection apparatus 10 of FIG. 1 described hereinabove are denoted by like reference characters, and description of them is suitably omitted.

In particular, in FIG. 22, in comparison with FIG. 1, the reference transmission plate 151 is provided in place of the reference reflector plate 101, and in addition to the sensor 103-1 and the exposure controlling unit 104-1 provided on the system of the lens 102 side, the sensor 103-2 and the exposure controlling unit 104-2 are provided also on the system of the reference transmission plate 151 side. It is to be noted that, as described hereinabove, the lens 102 and the sensor 103-1 are provided on the optical axis La. Meanwhile, the reference transmission plate 151 and the sensor 103-2 are provided on the optical axis La.

In FIG. 22, the reference transmission plate 151 is configured, for example, from a diffuse plate (diffuser plate) or the like. For example, the reference transmission plate 151 has an arbitrary transmittance. It is to be noted that the reference transmission plate 151 may be configured from a plurality of transmission plates for individual wavelengths to be measured.

The sensor 103-1 detects light (reflection light) from the inspection object 50 incident thereto through the lens 102 and outputs a measurement signal (measurement value) obtained as a result of the detection similarly to the sensor 103 (FIG. 1). The exposure controlling unit 104-1 controls the components of the lens 102 and the sensor 103-1 to perform exposure control similarly to the exposure controlling unit 104 (FIG. 1).

The sensor 103-2 detects light (transmission light) transmitted by the reference transmission plate 151 and outputs a measurement signal (measurement value) obtained as a result of the transmission similarly to the sensor 103 (FIG. 1). The exposure controlling unit 104-2 controls the components of the sensor 103-2 to perform exposure control similarly to the exposure controlling unit 104 (FIG. 1).

The correction processing unit 105 performs a correction process for correcting the spectral ratio (R/IR ratio) between the R channel value and the IR channel value on the basis of the measurement signals (measurement values) supplied thereto from the sensor 103-1 and the sensor 103-2 and supplies information indicative of the spectral ratio (R/IR ratio) after corrected to the inspection index calculation unit 106.

The inspection index calculation unit 106 uses the information supplied thereto from the correction processing unit 105 and indicative of the measurement spectral ratio (R/IR ratio) of the inspection object 50 after corrected to calculate an inspection index (NDVI value) of the inspection object 50 and outputs the inspection index (NDVI value).

The vegetation inspection apparatus 12 is configured in such a manner as described above.

Here, a processes executed by this vegetation inspection apparatus 12 is described particularly. In particular, upon measurement of an inspection index (NDVI value) of the inspection object 50 under a measurement light source, the vegetation inspection apparatus 12 refers to a lookup table (LUT) created in advance to determine a correction gain (measurement light source correspondence correction gain) of a spectral ratio according to a measurement spectral ratio (R/IR ratio) of the reference transmission plate 151 obtained by measurement under the measurement light source. Then, the vegetation inspection apparatus 12 uses the measurement light source correspondence correction gain to correct the measurement spectral ratio (R/IR ratio) of the inspection object 50 obtained by the measurement under the measurement light source and measures the inspection index (NDVI value) of the inspection object 50.

However, the lookup table (LUT) used here is a lookup table (LUT) that is created by the lookup table creation process described hereinabove and corresponds to the reference transmission plate 151, and is stored in advance in the storage unit 112 of the correction processing unit 105.

Further, here, measurement of the inspection index (NDVI value) of the inspection object 50 under the measurement light source is performed similarly to the measurement of the inspection index (NDVI value) of the inspection object 50 under the measurement light source described hereinabove with reference to FIGS. 10 to 12 or FIGS. 13 and 14. In this manner, the vegetation inspection apparatus 12 performs correction based on the correction gain (measurement light source correspondence correction gain) determined using the lookup table (LUT) corresponding to the reference transmission plate 151 and can thereby remove the light source dependency upon measurement of the inspection index (NDVI value) of the inspection object 50 under the measurement light source.

It is to be noted that, since, in the vegetation inspection apparatus 12, a plurality of sensors, namely, the sensor 103-1 and the sensor 103-2, are provided in the same housing, a dispersion may occur in spectral characteristic such as the sensitivity ratio between the R channel and the IR channel among different sensors. Therefore, preferably the vegetation inspection apparatus 12 adopts the configuration depicted in FIG. 13 such that it calculates in advance a spectral ratio for normalization taking, for example, the spectral characteristic of the reference light source and the spectral characteristic of the sensor 103-1 and the sensor 103-2 into consideration. This makes it possible in the vegetation inspection apparatus 12 to normalize the reference light source and the components of the sensors by dividing the measurement spectral ratio after correction by the spectral ratio for normalization calculated in advance, and a dispersion in spectral characteristic among the sensors can be suppressed.

(2) Compound Eye Configuration

(Configuration of vegetation inspection apparatus)

FIG. 23 is a perspective view illustrating an example of a configuration of an appearance of the vegetation inspection apparatus where the compound eye configuration is adopted.

Referring to FIG. 23, the vegetation inspection apparatus 13 is configured from a measurement unit 14, another measurement unit 15 and a processing unit 16. Further, in the vegetation inspection apparatus 13, the measurement unit 14 and the processing unit 16 are connected to each other and the measurement unit 15 and the processing unit 16 are connected to each other individually through a predetermined interface.

In FIG. 23, the measurement unit 14 has formed at a left side face side of a housing thereof a light incidence section 14A having a cylindrical shape that is hollow in the inside thereof such that light enters through the light incidence section 14A. Further, a sensor 103-1 is provided in the inside of the measurement unit 14.

In particular, light (reflection light) from the light incidence section 14A side enters the measurement unit 14 along an optical axis Lc and is received by the sensor face of the sensor 103-1. The sensor 103-1 measures the R channel value and the IR channel value in response to light (reflection light) from the inspection object 50 and outputs measurement values obtained by the measurement to the processing unit 16 through a predetermined interface.

The measurement unit 15 has formed at an upper face side of the housing thereof a light incidence section 15A that has a cylindrical shape that is hollow in the inside thereof such that light enters through the light incidence section 15A. Further, a sensor 103-2 is provided in the inside of the measurement unit 15. In particular, light (sun light) from the light incidence section 15A enters the measurement unit 15 along an optical axis Ld.

Here, in the inside of a circle C represented by a broken line of FIG. 23, part of the measurement unit 15 at the light incidence section 15A side is depicted in an enlarged scale. As depicted herein, a reference transmission plate 171 is provided on the optical axis Ld in the inside of the light incidence section 15A having a cylindrical shape.

In particular, in the measurement unit 15, light (sun light) from the light incidence section 15A side is transmitted through the reference transmission plate 171, and the transmission light is received by the sensor face of the sensor 103-2. Then, the sensor 103-2 measures the R channel value and the IR channel value according to the light (transmission light) transmitted through the reference transmission plate 171 and outputs measurement values obtained by the measurement to the processing unit 16 through a predetermined interface.

The processing unit 16 is configured, for example, from an FPGA (Field Programmable Gate Array), a personal computer or the like. To the processing unit 16, measurement values are inputted from the measurement unit 14 and the measurement unit 15 through a predetermined interface. The processing unit 16 determines an inspection index (NDVI value) of the inspection object 50 on the basis of the measurement values from the measurement unit 14 and the measurement unit 15.

Since the vegetation inspection apparatus 13 has a compound eye configuration as described above, reflection light reflected by the inspection object 50 and transmission light transmitted through the reference transmission plate 171 are measured by the sensor 103-1 and the sensor 103-2 provided in the housings different from each other, respectively. Now, a detailed internal configuration of the vegetation inspection apparatus 13 depicted in FIG. 23 is described with reference to FIG. 24.

Referring to FIG. 24, the vegetation inspection apparatus 13 is configured from a measurement unit 14, another measurement unit 15 and a processing unit 16. The measurement unit 14 is configured from a lens 102, a sensor 103-1, an exposure controlling unit 104-1 and an I/F unit 172-1. Meanwhile, the measurement unit 15 is configured from a reference transmission plate 171, a sensor 103-2, an exposure controlling unit 104-2 and an I/F unit 172-2. Further, the processing unit 16 is configured from a correction processing unit 105 and an inspection index calculation unit 106.

It is to be noted that, in the vegetation inspection apparatus 12 of FIG. 24, like portions to the blocks configuring the vegetation inspection apparatus 10 described hereinabove with reference to FIG. 1 are denoted by like reference characters, and description of them is suitably omitted. In particular, in comparison with FIG. 1, the components in FIG. 24 are arranged separately as the measurement unit 14, measurement unit 15 and processing unit 16, and the I/F unit 172-1 and the I/F unit 172-2 are provided at connection locations between the measurement unit 14 and measurement unit 15 and the processing unit 16, respectively.

Further, in FIG. 24, in comparison with FIG. 1, the reference transmission plate 171 is provided in place of the reference reflector plate 101, and separately from the sensor 103-1 and the exposure controlling unit 104-1 provided in the system of the lens 102 side, the sensor 103-2 and the exposure controlling unit 104-2 are provided also in the system of the reference transmission plate 171 side. It is to be noted that the lens 102 and the sensor 103-1 are provided on the optical axis Lc as described hereinabove. Further, the reference transmission plate 171 and the sensor 103-2 are provided on the optical axis Ld.

In the measurement unit 14, the sensor 103-1 detects light (reflection light) from the inspection object 50 incident through the lens 102 and outputs a measurement value obtained as a result of the detection. The exposure controlling unit 104-1 performs exposure control by controlling the components of the lens 102 and the sensor 103-1. The I/F unit 172-1 outputs the measurement value from the sensor 103-1 to the processing unit 16.

In the measurement unit 15, the sensor 103-2 detects light (transmission light) transmitted through the reference transmission plate 171 and outputs a measurement value obtained as a result of the detection. The exposure controlling unit 104-2 performs exposure control by controlling the components of the sensor 103-2. The I/F unit 172-2 outputs the measurement value from the sensor 103-2 to the processing unit 16.

In the processing unit 16, the measurement value from the I/F unit 172-1 of the measurement unit 14 and the measurement value from the I/F unit 172-2 of the measurement unit 15 are inputted to the correction processing unit 105.

The correction processing unit 105 performs a correction process for correcting the spectral ratio (R/IR ratio) between the R channel value and the IR channel value on the basis of the measurement values inputted from the measurement unit 14 and the measurement unit 15, and supplies information indicative of the spectral ratio (R/IR ratio) after corrected to the inspection index calculation unit 106.

The inspection index calculation unit 106 uses the information supplied thereto from the correction processing unit 105 and indicative of the measurement spectral ratio (R/IR ratio) of the inspection object 50 to calculate an inspection index (NDVI value) of the inspection object 50 and outputs the inspection index (NDVI value).

The vegetation inspection apparatus 13 is configured in such a manner as described above.

Here, a process executed by the vegetation inspection apparatus 13 is described particularly. In particular, upon measurement of the inspection index (NDVI value) of the inspection object 50 under the measurement light source, the vegetation inspection apparatus 13 refers to the lookup table (LUT) created in advance to determine a correction gain (measurement light source correspondence correction gain) of the spectral ratio according to the measurement spectral ratio (R/IR ratio) of the reference transmission plate 171 obtained by the measurement under the measurement light source. Then, the vegetation inspection apparatus 13 uses the measurement light source correspondence correction gain to correct the measurement spectral ratio (R/IR ratio) of the inspection object 50 obtained by the measurement under the measurement light source, and measures the inspection index (NDVI value) of the inspection object 50.

However, the lookup table (LUT) used here is a lookup table (LUT) that is created by the lookup table creation process described above and corresponds to the reference transmission plate 171, and is stored in advance in the storage unit 112 of the correction processing unit 105.

Further, here, measurement of the inspection index (NDVI value) of the inspection object 50 under the measurement light source is performed similarly to the measurement of the inspection index (NDVI value) of the inspection object 50 under the measurement light source described hereinabove with reference to FIGS. 10 to 12 or FIGS. 13 and 14. In this manner, the vegetation inspection apparatus 13 performs correction based on the correction gain (measurement light source correspondence correction gain) determined using the lookup table (LUT) corresponding to the reference transmission plate 171 and can thereby remove the light source dependency upon measurement of the inspection index (NDVI value) of the inspection object 50 under the measurement light source.

It is to be noted that, in the vegetation inspection apparatus 13, since a plurality of sensors including the sensor 103-1 and the sensor 103-2 are provided in different housings, it is supposed that a dispersion occurs in spectral characteristic among sensors similarly as in the vegetation inspection apparatus 12. Therefore, also in the vegetation inspection apparatus 13, by adopting the configuration depicted in FIG. 13 such that a spectral ratio for normalization is calculated in advance similarly in the vegetation inspection apparatus 12, a dispersion in spectral characteristic among the sensors can be suppressed.

<6. Configuration of Computer>

While the series of processes described above can be executed by hardware, it may otherwise be executed by software. Where the series of processes is executed by software, a program that constructs the software is installed into a computer. FIG. 25 is a view depicting an example of a configuration of hardware of a computer that executes the series of processes described hereinabove in accordance with a program.

In the computer 900, a CPU (Central Processing Unit) 901, a ROM (Read Only Memory) 902 and a RAM (Random Access Memory) 903 are connected to each other by a bus 904. To the bus 904, an input/output interface 905 is connected further. To the input/output interface 905, an inputting unit 906, an outputting unit 907, a recording unit 908, a communication unit 909 and a drive 910 are connected.

The inputting unit 906 is configured from a keyboard, a mouse, a microphone and so forth. The outputting unit 907 is configured from a display unit, a speaker and so forth. The recording unit 908 is configured from a hard disk, a nonvolatile memory and so forth. The communication unit 909 is configured from a network interface and so forth. The drive 910 drives a removable storage medium 911 such as a magnetic disk, an optical disk, a magneto-optical disk or a semiconductor memory.

In the computer 900 configured in such a manner as described above, the CPU 901 loads a program recorded, for example, in the ROM 902 or the recording unit 908 into the RAM 903 through the input/output interface 905 and the bus 904 and executes the program to perform the series of processes described hereinabove.

The program executed by the computer 900 (CPU 901) can be recorded on and provided as the removable storage medium 911, for example, as a package storage medium or the like. Further, the program can be provided through a wired or wireless transmission medium such as a local area network, the Internet or a digital satellite broadcast.

In the computer 900, the program can be installed into the recording unit 908 through the input/output interface 905 by loading the removable storage medium 911 into the drive 910. Alternatively, the program can be received by the communication unit 909 through a wired or wireless transmission medium and installed into the recording unit 908. Alternatively, the program may be installed in advance into the ROM 902 or the recording unit 908.

It is to be noted that, in the present specification, the processes performed in accordance with the program by the computer may not necessarily be performed in a time series in accordance with an order described as the flow charts. In other words, the processes performed in accordance with the program by the computer include also processes executed in parallel or individually (for example, parallel processing or processing by objects). Further, the program may be processed by one computer (processor) or may be processed by distributed processing by a plurality of computers.

It is to be noted that the embodiment of the present technology is not limited to the embodiment described hereinabove but can be altered in various manners without departing from the subject matter of the present technology. For example, it is possible to adopt a form that includes all or part of a plurality of embodiments described hereinabove.

Further, the present technology can take such configurations as described below.

(1) An inspection apparatus, including:

a correction gain determination unit configured to determine, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and

a correction unit configured to correct the measurement spectral information of the inspection object obtained by sensing under the measurement light source based on the determined measurement light source correspondence correction gain.

(2) The inspection apparatus according to (1), in which
the reference spectral information is reference spectral information of the reference reflector plate or the reference transmission plate obtained by sensing the reference reflector plate or the reference transmission plate under a plurality of assumed light sources assumed in advance.

(3) The inspection apparatus according to (2), in which
the plurality of assumed light sources include a reference light source that serves as a reference, and
the correction gain associated with the reference spectral information of the reference reflector plate or the reference transmission plate for each of the assumed light sources is associated with the correction gain of the reference light source.

(4) The inspection apparatus according to any one of (1) to (3), in which
the reference spectral information of the reference reflector plate or the reference transmission plate is a reference spectral ratio of the reference reflector plate or the reference transmission plate,
the measurement spectral information of the reference reflector plate or the reference transmission plate is a measurement spectral ratio of the reference reflector plate or the reference transmission plate, and
the measurement spectral information of the inspection object is a measurement spectral ratio of the inspection object.

(5) The inspection apparatus according to (4), further including:
an inspection index calculation unit configured to calculate an inspection index of the inspection object based on the corrected measurement spectral ratio of the inspection object.

(6) The inspection apparatus according to (5), further including:
a display controlling unit configured to control display of an image corresponding to the inspection index of the inspection object.

(7) The inspection apparatus according to any one of (1) to (6), in which
the correction gain is calculated using a representative spectral characteristic of a plurality of inspection objects having characteristics different from each other.

(8) The inspection apparatus according to (7), in which
the representative spectral characteristic is an average spectral characteristic of spectral characteristics of the plurality of inspection objects having characteristics different from each other.

(9) The inspection apparatus according to (7) or (8), in which
the correction gain is calculated using known spectral characteristics of the assumed light sources and a known spectral sensitivity characteristic of a sensor unit.

(10) The inspection apparatus according to (4), in which
the reference spectral ratio of the reference reflector plate is calculated using a known spectral characteristic of an arbitrary reference reflector plate.

(11) The inspection apparatus according to any one of (1) to (10), in which
the correction gain determination unit selects the measurement light source correspondence correction gain from among a plurality of correction gains stored in the table.

(12) The inspection apparatus according to any one of (1) to (10), in which
the correction gain determination unit calculates the measurement light source correspondence correction gain by performing interpolation using values of a plurality of correction gains stored in the table.

(13) The inspection apparatus according to any one of (1) to (12), further including:

a storage unit configured to store the table for each of inspection objects of one kind or a plurality of kinds, in which

the correction gain determination unit refers to the table according to a type of the inspection object from among the tables stored in the storage unit to determine the measurement light source correspondence correction gain.

(14) The inspection apparatus according to any one of (1) to (13), further including:

a sensor unit configured to sense, when the reference reflector plate is used from between the reference reflector plate and the reference transmission plate, the inspection object and the reference reflector plate under the measurement light source.

(15) The inspection apparatus according to (14), in which

the sensor unit senses the inspection object and the reference reflector plate simultaneously.

(16) The inspection apparatus according to (14) or (15), in which

the reference reflector plate is attached to a given position so as to exist in an angle of view of a camera the sensor unit has.

(17) The inspection apparatus according to any one of (4) to (16), in which

the inspection object is a plant,

each of the reference spectral ratio of the reference reflector plate or the reference transmission plate, the measurement spectral ratio of the reference reflector plate or the reference transmission plate and the measurement spectral ratio of the inspection object is a ratio between a value of an R (red) component and a value of an IR (infrared) component, and

the inspection index is a normalized vegetation index (NDVI: Normalized Difference Vegetation Index).

(18) The inspection apparatus according to any one of (1) to (4), further including:

where the reference transmission plate is used from between the reference reflector plate and the reference transmission plate, a first sensor unit configured to sense the inspection object under the measurement light source and a second sensor unit configured to sense the reference transmission plate under the measurement light source.

(19) An inspection method, including the steps of:

determining, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and

correcting the measurement spectral information of the inspection object obtained by sensing under the measurement light source based on the determined measurement light source correspondence correction gain.

(20) A program for causing a computer to function as:

a correction gain determination unit configured to determine, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and

a correction unit configured to correct the measurement spectral information of the inspection object obtained by sensing under the measurement light source based on the determined measurement light source correspondence correction gain.

(21) A table creation apparatus, including:

a first spectral information calculation unit configured to calculate reference spectral information;

a correction gain calculation unit configured to calculate a correction gain for correcting measurement spectral information of an inspection object obtained by sensing under a measurement light source; and

a table creation unit configured to create a table in which the reference spectral information and the correction gain are associated with each other.

(22) The table creation apparatus according to (21), further including:

a second spectral information calculation unit configured to calculate assumed spectral information of the inspection object under a plurality of assumed light sources assumed in advance, in which

the first spectral information calculation unit calculates assumed spectral information of a reference reflector plate or a reference transmission plate under the plurality of assumed light sources, and
the table creation unit creates a table in which, for each of the plurality of assumed light sources, assumed spectral information of the reference reflector plate or the reference transmission plate as the reference spectral information and the correction gain are associated with each other.

(23) The table creation apparatus according to (22), in which
the assumed spectral information of the reference reflector plate or the reference transmission plate is an assumed spectral ratio of the reference reflector plate and the reference transmission plate,
the assumed spectral information of the inspection object is an assumed spectral ratio of the inspection object, and
the measurement spectral information of the inspection object is a measurement spectral ratio of the inspection object.
(24) A table creation method, including the steps of:

calculating reference spectral information;
calculating a correction gain for correcting measurement spectral information of an inspection object obtained by sensing under a measurement light source; and
creating a table in which the reference spectral information and the correction gain are associated with each other.

(25) A program for causing a computer to function as:

a first spectral information calculation unit configured to calculate reference spectral information;
a correction gain calculation unit configured to calculate a correction gain for correcting measurement spectral information of an inspection object obtained by sensing under a measurement light source; and
a table creation unit configured to create a table in which the reference spectral information and the correction gain are associated with each other.

[Reference Signs List]

10, 12, 13 Vegetation inspection apparatus, 11 Vegetation inspection system, 14, 15 Measurement unit, 16 Processing unit, 20 Lookup table creation apparatus, 60 Measurement apparatus, 60A Fixed point measurement apparatus, 60B Moving measurement apparatus, 60C Satellite measurement apparatus, 70 Processing apparatus, 80 Storage apparatus, 101 Reference reflector plate, 102 Lens, 103, 103-1, 103-2 Sensor, 104, 104-1, 104-2 Exposure controlling unit, 105 Correction processing unit, 106 Inspection index calculation unit, 111 Correction gain processing unit, 112 Storage unit, 113 Measurement light correction unit, 121 Reference reflector plate R/IR ratio calculation unit, 122 Correction gain determination unit, 123 Inspection object R/IR ratio calculation unit, 124 Gain correction unit, 131 NDVI value calculation unit, 132 Normalization unit, 133 Storage unit, 151, 171 Reference transmission plate, 201 Inspection object average spectral characteristic calculation unit, 202 Reference reflector plate measurement sensor output value calculation unit, 203 Reference reflector plate R/IR ratio calculation unit, 204 Inspection object measurement sensor output value calculation unit, 205 Inspection object R/IR ratio calculation unit, 206 Correction gain calculation unit, 207 Lookup table creation unit, 900 Computer, 901 CPU

**Claims**

1. An inspection apparatus, comprising:

   a correction gain determination unit configured to determine, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and
   a correction unit configured to correct the measurement spectral information of the inspection object obtained by sensing under the measurement light source based on the determined measurement light source correspondence correction gain.

2. The inspection apparatus according to claim 1, wherein

the reference spectral information is reference spectral information of the reference reflector plate or the reference transmission plate obtained by sensing the reference reflector plate or the reference transmission plate under a plurality of assumed light sources assumed in advance.

3. The inspection apparatus according to claim 2, wherein
the plurality of assumed light sources include a reference light source that serves as a reference, and
the correction gain associated with the reference spectral information of the reference reflector plate or the reference transmission plate for each of the assumed light sources is associated with the correction gain of the reference light source.

4. The inspection apparatus according to claim 2, wherein
the reference spectral information of the reference reflector plate or the reference transmission plate is a reference spectral ratio of the reference reflector plate or the reference transmission plate,
the measurement spectral information of the reference reflector plate or the reference transmission plate is a measurement spectral ratio of the reference reflector plate or the reference transmission plate, and
the measurement spectral information of the inspection object is a measurement spectral ratio of the inspection object.

5. The inspection apparatus according to claim 4, further comprising:
an inspection index calculation unit configured to calculate an inspection index of the inspection object based on the corrected measurement spectral ratio of the inspection object.

6. The inspection apparatus according to claim 5, further comprising:
a display controlling unit configured to control display of an image corresponding to the inspection index of the inspection object.

7. The inspection apparatus according to claim 1, wherein
the correction gain is calculated using a representative spectral characteristic of a plurality of inspection objects having characteristics different from each other.

8. The inspection apparatus according to claim 7, wherein
the representative spectral characteristic is an average spectral characteristic of spectral characteristics of the plurality of inspection objects having characteristics different from each other.

9. The inspection apparatus according to claim 7, wherein
the correction gain is calculated using known spectral characteristics of the assumed light sources and a known spectral sensitivity characteristic of a sensor unit.

10. The inspection apparatus according to claim 4, wherein
the reference spectral ratio of the reference reflector plate is calculated using a known spectral characteristic of an arbitrary reference reflector plate.

11. The inspection apparatus according to claim 1, wherein
the correction gain determination unit selects the measurement light source correspondence correction gain from among a plurality of correction gains stored in the table.

12. The inspection apparatus according to claim 1, wherein
the correction gain determination unit calculates the measurement light source correspondence correction gain by performing interpolation using values of a plurality of correction gains stored in the table.

13. The inspection apparatus according to claim 1, further comprising:

a storage unit configured to store the table for each of inspection objects of one kind or a plurality of kinds, wherein the correction gain determination unit refers to the table according to a type of the inspection object from among the tables stored in the storage unit to determine the measurement light source correspondence correction gain.

14. The inspection apparatus according to claim 1, further comprising:
a sensor unit configured to sense, when the reference reflector plate is used from between the reference reflector plate and the reference transmission plate, the inspection object and the reference reflector plate under the measurement light source.

**15.** The inspection apparatus according to claim 14, wherein
the sensor unit senses the inspection object and the reference reflector plate simultaneously.

**16.** The inspection apparatus according to claim 14, wherein
the reference reflector plate is attached to a given position so as to exist in an angle of view of a camera the sensor unit has.

**17.** The inspection apparatus according to claim 4, wherein
the inspection object is a plant,
each of the reference spectral ratio of the reference reflector plate or the reference transmission plate, the measurement spectral ratio of the reference reflector plate or the reference transmission plate and the measurement spectral ratio of the inspection object is a ratio between a value of an R (red) component and a value of an IR (infrared) component, and
the inspection index is a normalized vegetation index (NDVI: Normalized Difference Vegetation Index).

**18.** The inspection apparatus according to claim 1, further comprising:
where the reference transmission plate is used from between the reference reflector plate and the reference transmission plate, a first sensor unit configured to sense the inspection object under the measurement light source and a second sensor unit configured to sense the reference transmission plate under the measurement light source.

**19.** An inspection method, comprising the steps of:

determining, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and
correcting the measurement spectral information of the inspection object obtained by sensing under the measurement light source based on the determined measurement light source correspondence correction gain.

**20.** A program for causing a computer to function as:

a correction gain determination unit configured to determine, based on a table in which reference spectral information and a correction gain for correcting measurement spectral information of an inspection object obtained by sensing under a measurement light source are associated with each other and measurement spectral information of a reference reflector plate or a reference transmission plate obtained by sensing under the measurement light source, a measurement light source correspondence correction gain corresponding to the measurement light source; and
a correction unit configured to correct the measurement spectral information of the inspection object obtained by sensing under the measurement light source based on the determined measurement light source correspondence correction gain.

## FIG.1

EP 3 321 661 A1

# F I G . 2

SPECTRAL REFLECTANCE CHARACTERISTIC OF INSPECTION OBJECT

# FIG.3

SPECTRAL SENSITIVITY CHARACTERISTIC OF SENSOR

- - - - - - - : Rch VALUE

———— : IRch VALUE

SPECTRAL CHARACTERISTICS WHERE REFERENCE REFLECTOR PLATE OF FLAT SPECTRAL REFLECTANCE IS
USED TO DIRECTLY DETERMINE CORRECTION GAIN TO PERFORM MEASUREMENT LIGHT CORRECTION

ASSUMED LIGHT SOURCE L1    ASSUMED LIGHT SOURCE L2

(A) SENSOR SPECTRAL SENSITIVITY CHARACTERISTIC $S(\lambda)$

(B) LIGHT SOURCE SPECTRAL CHARACTERISTIC $I(\lambda)$

(C) MEASUREMENT SYSTEM OVERALL SPECTRAL CHARACTERISTIC $S(\lambda) \times I(\lambda)$

(D) SPECTRAL REFLECTANCE CHARACTERISTIC OF REFERENCE REFLECTOR PLATE $O\_Ref(\lambda)$

(E) REFERENCE REFLECTOR PLATE SYSTEM OVERALL SPECTRAL CHARACTERISTIC
$S(\lambda) \times I(\lambda) \times O\_Ref(\lambda)$

1.0  1.0      0.25  1.0

(F) SPECTRAL REFLECTANCE CHARACTERISTIC OF INSPECTION OBJECT $O\_ISP(\lambda)$

(G) INSPECTION OBJECT SYSTEM OVERALL SPECTRAL CHARACTERISTIC
$S(\lambda) \times I(\lambda) \times O\_ISP(\lambda)$

0.25  1.0      0.05  1.0

FIG.4

SPECTRAL CHARACTERISTICS WHERE LOOKUP TABLE IS USED TO PERFORM MEASUREMENT LIGHT CORRECTION

ASSUMED LIGHT SOURCE L1    ASSUMED LIGHT SOURCE L2

(A) SENSOR SPECTRAL SENSITIVITY CHARACTERISTIC $S(\lambda)$

(B) LIGHT SOURCE SPECTRAL CHARACTERISTIC $I(\lambda)$

(C) MEASUREMENT SYSTEM OVERALL SPECTRAL CHARACTERISTIC $S(\lambda) \times I(\lambda)$

(D) SPECTRAL REFLECTANCE CHARACTERISTIC OF REFERENCE REFLECTOR PLATE $O\_Ref(\lambda)$

(E) REFERENCE REFLECTOR PLATE SYSTEM OVERALL SPECTRAL CHARACTERISTIC
$S(\lambda) \times I(\lambda) \times O\_Ref(\lambda)$

1.0   1.0        0.25   1.0

(H) SPECTRAL REFLECTANCE REPRESENTATIVE CHARACTERISTIC OF INSPECTION OBJECT
$O\_Plt\_AVE(\lambda)$

(I) INSPECTION OBJECT SYSTEM OVERALL SPECTRAL REPRESENTATIVE CHARACTERISTIC
$S(\lambda) \times I(\lambda) \times O\_Plt\_AVE(\lambda)$

0.25   1.0        0.05   1.0

MEASUREMENT LIGHT SOURCE L1    MEASUREMENT LIGHT SOURCE L2

(F) SPECTRAL REFLECTANCE CHARACTERISTIC OF INSPECTION OBJECT $O\_Plt\_ISP(\lambda)$

(G) INSPECTION OBJECT SYSTEM OVERALL SPECTRAL CHARACTERISTIC
$S(\lambda) \times I(\lambda) \times O\_Plt\_ISP(\lambda)$

0.25   1.0        0.05   1.0

FIG.5

EP 3 321 661 A1

FIG.6

EP 3 321 661 A1

# F I G . 7

START OF LOOKUP TABLE CREATION PROCESS

↓

MEASURE OR CALCULATE KNOWN SPECTRAL INFORMATION
$I\_2800K(\lambda)$, $I\_5500K(\lambda)$, $I\_10000K(\lambda)$ — S11

↓

MEASURE KNOWN SPECTRAL SENSITIVITY CHARACTERISTIC OF EACH CHANNEL OF MEASUREMENT APPARATUS
$S\_R(\lambda)$, $S\_IR(\lambda)$ — S12

↓

MEASURE SPECTRAL REFLECTANCE CHARACTERISTIC $O\_Ref(\lambda)$ OF REFERENCE REFLECTOR PLATE — S13

↓

MEASURE SPECTRAL REFLECTANCE CHARACTERISTIC $O\_Plt\_i(\lambda)$ OF
PLURAL INSPECTION OBJECTS OF DIFFERENT CHARACTERISTICS — S14

↓

CALCULATE AVERAGE SPECTRAL REFLECTANCE CHARACTERISTIC $O\_Plt\_AVE(\lambda)$
OF SPECTRAL REFLECTANCE CHARACTERISTIC $O\_Plt\_i(\lambda)$ — S15

↓

CALCULATE Rch VALUE AND IRch VALUE WHEN REFERENCE REFLECTOR
PLATE IS MEASURED UNDER EACH ASSUMED LIGHT SOURCE
$D\_Ref\_2800K\_R$, $D\_Ref\_5500K\_R$, $D\_Ref\_10000K\_R$
$D\_Ref\_2800K\_IR$, $D\_Ref\_5500K\_IR$, $D\_Ref\_10000K\_IR$ — S16

↓

CALCULATE ASSUMED SPECTRAL RATIO OF REFERENCE REFLECTOR PLATE UNDER EACH ASSUMED LIGHT SOURCE
$R\_Ref\_2800K = D\_Ref\_2800K\_R / D\_Ref\_2800K\_IR$
$R\_Ref\_5500K = D\_Ref\_5500K\_R / D\_Ref\_5500K\_IR$
$R\_Ref\_10000K = D\_Ref\_10000K\_R / D\_Ref\_10000K\_IR$ — S17

↓

CALCULATE Rch VALUE AND IRch VALUE WHEN INSPECTION OBJECT IS
MEASURED UNDER EACH ASSUMED LIGHT SOURCE
$D\_Plt\_AVE\_2800K\_R$, $D\_Plt\_AVE\_5500K\_R$, $D\_Plt\_AVE\_10000K\_R$
$D\_Plt\_AVE\_2800K\_IR$, $D\_Plt\_AVE\_5500K\_IR$, $D\_Plt\_AVE\_10000K\_IR$ — S18

↓

CALCULATE ASSUMED SPECTRAL RATIO OF INSPECTION OBJECT UNDER EACH ASSUMED LIGHT SOURCE
$R\_Plt\_AVE\_2800K = D\_Plt\_AVE\_2800K\_R / D\_Plt\_AVE\_2800K\_IR$
$R\_Plt\_AVE\_5500K = D\_Plt\_AVE\_5500K\_R / D\_Plt\_AVE\_5500K\_IR$
$R\_Plt\_AVE\_10000K = D\_Plt\_AVE\_10000K\_R / D\_Plt\_AVE\_10000K\_IR$ — S19

↓

CALCULATE CORRECTION GAIN FOR EACH ASSUMED LIGHT SOURCE
$G\_Plt\_AVE\_2800K = R\_Plt\_AVE\_5500K / R\_Plt\_AVE\_2800K$
$G\_Plt\_AVE\_5500K = R\_Plt\_AVE\_5500K / R\_Plt\_AVE\_5500K$
$G\_Plt\_AVE\_10000K = R\_Plt\_AVE\_5500K / R\_Plt\_AVE\_10000K$ — S20

↓

CREATE LOOKUP TABLE — S21

↓

END

EXAMPLE OF ASSUMED LIGHT SOURCES

ASSUMED LIGHT SOURCES OF THREE DIFFERENT COLOR TEMPERATURES

- - - - - - - : 2800K
(ASSUMED LIGHT SOURCE)

————— : 5500K
(ASSUMED LIGHT SOURCE→REFERENCE LIGHT SOURCE)

— - — - — : 10000K
(ASSUMED LIGHT SOURCE)

FIG.8

# F I G . 9

| ASSUMED LIGHT SOURCE (COLOR TEMPERATURE) | REFERENCE SPECTRAL RATIO OF REFERENCE REFLECTOR PLATE | CORRECTION GAIN |
|---|---|---|
| 2800K | R_Ref_2800K | G_Plt_AVE_2800K |
| 5500K | R_Ref_5500K | G_Plt_AVE_5500K |
| 10000K | R_Ref_10000K | G_Plt_AVE_10000K |

# FIG.10

# FIG.11

```
┌─────────────────────────────────┐
│     START OF FIRST MEASUREMENT   │
│     PROCESS OF INSPECTION INDEX  │
└─────────────────────────────────┘
```

MEASURE Rch VALUE AND IRch VALUE OF REFERENCE
REFLECTOR PLATE UNDER MEASUREMENT LIGHT SOURCE
D_Ref_env_R, D_Ref_env_IR — S111

CALCULATE MEASUREMENT SPECTRAL RATIO OF REFERENCE REFLECTOR PLATE
R_Ref_env=D_Ref_env_R/D_Ref_env_IR — S112

REFER TO LOOKUP TABLE (LUT) TO SEARCH FOR ASSUMED LIGHT SOURCE OF
COLOR TEMPERATURE AT WHICH REFERENCE SPECTRAL RATIO OF REFERENCE
REFLECTOR PLATE IS CLOSEST TO MEASUREMENT SPECTRAL RATIO OF REFERENCE
REFLECTOR PLATE CALCULATED FROM AMONG PLURAL MEASUREMENT LIGHT
SOURCES AND DETERMINE ASSUMED LIGHT SOURCE AS ESTIMATED LIGHT SOURCE — S113

REFER TO LOOKUP TABLE (LUT) TO DETERMINE MEASUREMENT
LIGHT SOURCE CORRESPONDENCE CORRECTION GAIN
G_Plt_AVE_env — S114

MEASURE Rch VALUE AND IRch VALUE OF INSPECTION
OBJECT UNDER MEASUREMENT LIGHT SOURCE
D_Plt_ISP_env_R, D_Plt_ISP_env_IR — S115

CALCULATE MEASUREMENT SPECTRAL RATIO OF INSPECTION OBJECT
R_Plt_ISP_env=D_Plt_ISP_env_R/D_Plt_ISP_env_IR — S116

CORRECT MEASUREMENT SPECTRAL RATIO OF INSPECTION OBJECT
R_Plt_ISP_env_comp=R_Plt_ISP_env × G_Plt_AVE_env — S117

CALCULATE NDVI VALUE
NDVI=(1−R_Plt_ISP_env_comp)/(1+R_Plt_ISP_env_comp) — S118

```
┌─────────┐
│   END   │
└─────────┘
```

# FIG.12

| ASSUMED LIGHT SOURCE (COLOR TEMPERATURE) | REFERENCE SPECTRAL RATIO OF REFERENCE REFLECTOR PLATE | CORRECTION GAIN |
|---|---|---|
| 2800K | 0. 25 | 5. 0 |
| 5500K | 1. 0 | 1. 0 |
| 10000K | 4. 0 | 0. 3 |

# FIG.13

# FIG.14

$$\text{START OF SECOND MEASUREMENT}$$
$$\text{PROCESS OF INSPECTION INDEX}$$

---

MEASURE Rch VALUE AND IRch VALUE OF REFERENCE
REFLECTOR PLATE UNDER MEASUREMENT LIGHT SOURCE
D_Ref_env_R, D_Ref_env_IR

S131

---

CALCULATE MEASUREMENT SPECTRAL RATIO OF REFERENCE REFLECTOR PLATE
R_Ref_env=D_Ref_env_R/D_Ref_env_IR

S132

---

REFER TO LOOKUP TABLE (LUT) TO SEARCH FOR ASSUMED LIGHT SOURCE OF
COLOR TEMPERATURE AT WHICH REFERENCE SPECTRAL RATIO OF REFERENCE
REFLECTOR PLATE IS CLOSEST TO MEASUREMENT SPECTRAL RATIO OF REFERENCE
REFLECTOR PLATE CALCULATED FROM AMONG PLURAL MEASUREMENT LIGHT
SOURCES AND DETERMINE ASSUMED LIGHT SOURCE AS ESTIMATED LIGHT SOURCE

S133

---

REFER TO LOOKUP TABLE (LUT) TO DETERMINE MEASUREMENT
LIGHT SOURCE CORRESPONDENCE CORRECTION GAIN
G_Plt_AVE_env

S134

---

MEASURE Rch VALUE AND IRch VALUE OF INSPECTION
OBJECT UNDER MEASUREMENT LIGHT SOURCE
D_Plt_ISP_env_R, D_Plt_ISP_env_IR

S135

---

CALCULATE MEASUREMENT SPECTRAL RATIO OF INSPECTION OBJECT
R_Plt_ISP_env=D_Plt_ISP_env_R/D_Plt_ISP_env_IR

S136

---

CORRECT MEASUREMENT SPECTRAL RATIO OF INSPECTION OBJECT
R_Plt_ISP_env_comp=R_Plt_ISP_env × G_Plt_AVE_env

S137

---

PERFORM NORMALIZATION OF LIGHT SOURCE-SENSOR COMPONENTS
R_Plt_ISP_norm=R_Plt_ISP_env_comp/R_Flat_5500K

S138

---

CALCULATE NDVI VALUE
NDVI = (1−R_Plt_ISP_norm)/(1+R_Plt_ISP_norm)

S139

---

$$\text{END}$$

# ＦＩＧ．１５

A

B

# FIG.16

EP 3 321 661 A1

# FIG.17

# FIG.18

# FIG.19

EP 3 321 661 A1

# FIG.20

A    60A    61A

B    61B    60B

C    60C    61C

F I G . 2 1

# FIG.22

EP 3 321 661 A1

EP 3 321 661 A1

F I G . 2 3

# FIG.24

EP 3 321 661 A1

# FIG.25

EP 3 321 661 A1

## INTERNATIONAL SEARCH REPORT

| International application No. |
| --- |
| PCT/JP2016/068781 |

**A.  CLASSIFICATION OF SUBJECT MATTER**
*G01N21/27(2006.01)i*

According to International Patent Classification (IPC) or to both national classification and IPC

**B.  FIELDS SEARCHED**

Minimum documentation searched (classification system followed by classification symbols)
G01N21/00, 21/01, 21/17-21/61, G01J3/00-3/52

Documentation searched other than minimum documentation to the extent that such documents are included in the fields searched
Jitsuyo Shinan Koho        1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)
JSTPlus/JMEDPlus/JST7580(JDreamIII)

**C.  DOCUMENTS CONSIDERED TO BE RELEVANT**

| Category* | Citation of document, with indication, where appropriate, of the relevant passages | Relevant to claim No. |
| --- | --- | --- |
| A | JP 2014-126529 A  (Horiba, Ltd.),<br>07 July 2014 (07.07.2014),<br>paragraphs [0014], [0015]<br>& US 2014/0185049 A1<br>paragraphs [0011], [0012]<br>& KR 10-2014-0085348 A  & TW 201425907 A | 1-20 |
| A | JP 5-264349 A  (Hughes Aircraft Co.),<br>12 October 1993 (12.10.1993),<br>paragraphs [0016], [0017]<br>& US 5340974 A<br>column 5, line 54 to column 6, line 33<br>& EP 558854 A1 | 1-20 |

☒  Further documents are listed in the continuation of Box C.          ☐  See patent family annex.

| | |
| --- | --- |
| *   Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| 07 September 2016 (07.09.16) | 20 September 2016 (20.09.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

International application No.

PCT/JP2016/068781

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2005-127980 A (JFE Advantech Co., Ltd.), 19 May 2005 (19.05.2005), paragraphs [0036] to [0042] (Family: none) | 1-20 |
| A | JP 2002-169351 A (Samsung Yokohama Research Institute), 14 June 2002 (14.06.2002), paragraphs [0012] to [0014] (Family: none) | 1-20 |

Form PCT/ISA/210 (continuation of second sheet) (January 2015)

**REFERENCES CITED IN THE DESCRIPTION**

**Patent documents cited in the description**

- JP 2006101768 A